# EUROPÄISCHE PATENTSCHRIFT

(11) **EP 0 879 257 B1**
(45) Veröffentlichungstag und Bekanntmachung des Hinweises auf die Patenterteilung: **03.04.2002**
(21) Anmeldenummer: 97951256.3
(22) Anmeldetag: 21.11.1997
(51) Int. Cl.: C08G 59/02, C08G 59/22, C08G 59/24, C08G 59/26, C08G 59/32, C08G 59/38, A61K 6/087, C09J 163/00

(54) **POLYMERISIERBARE MASSEN AUF DER BASIS VON EPOXIDEN**
POLYMERIZABLE SUBSTANCES BASED ON EPOXIDES
SUBSTANCES POLYMERISABLES A BASE D'EPOXIDES

(30) Priorität: 21.11.1996 DE 19648283
(43) Veröffentlichungstag der Anmeldung: 25.11.1998
(73) Patentinhaber: 3M ESPE AG, 82229 Seefeld (DE)
(72) Erfinder: WEINMANN, Wolfgang, D-82211 Herrsching (DE); GASSER, Oswald, D-82229 Seefeld (DE); GUGGENBERGER, Rainer, D-82211 Herrsching (DE); LECHNER, Günther, D-82237 Wörthsee (DE); SOGLOWEK, Wolfgang, D-82211 Herrsching (DE); ZECH, Joachim, D-82229 Hechendorf (DE)
(74) Vertreter: Freiherr von Wittgenstein, Arved, Dr.
(86) Internationale Anmeldenummer: EP9706504
(87) Internationale Veröffentlichungsnummer: WO9822521

(56) Entgegenhaltungen:
- EP-A- 0 394 192
- EP-A- 0 532 896
- WO-A-95/30402

## Beschreibung

Die Erfindung betrifft polymerisierbare Massen auf der Basis von Epoxiden, neue cycloaliphatische Epoxide und ihre Verwendung.

In polymerisierbaren Dentalmassen wurden bislang vorwiegend Methacrylat- und Acrylatmonomere verwendet. Besondere Aufmerksamkeit verdient das von Bowen beschriebene 2,2-Bis[4,1-phenylenoxy(2-hydroxy-3,1-propandiyl)-methacrylsäureester]-propyliden (Bis-GMA) [US-A-3 066 112]. Mischungen dieses Methacrylats mit Triethylenglykoldimethacrylat dienen auch heute noch als Standardrezeptur für dentale plastische Direkt-Füllungswerkstoffe. Auch Methacrylderivate des zweifach formylierten Bis-(hydroxymethyl)tricyclo[5.2.1.0^{2,6}]-decans haben sich als Monomere für Dentalcomposite bewährt [W. Gruber et al., DE-A-27 14 538; W. Schmitt et al., DE-C-28 16 823; J. Reiners et al., EP-A-0 261 520]. Ein großer Nachteil der bekannten polymerisierbaren Dentalmassen ist der Polymerisationsschrumpf, der beispielsweise bei der Anwendung als Füllungsmaterial durch die Bildung von Randspalten Sekundärkaries verursachen kann. Weiterhin führt bei Dentalmassen auf Acrylatbasis die Polymerisationsinhibierung durch Sauerstoff zur Ausbildung einer sogenannten Schmierschicht, die beispielsweise bei Füllungen unerwünscht, sogar schädlich ist. Schließlich weisen polymerisierbare Dentalmassen auf Acrylatbasis eine geringe Haftung an der Zahnsubstanz auf.

Obwohl es umfangreiche Erfahrungen mit Epoxiden und cycloaliphatischen Epoxiden gibt (US-A-2 716 123, US-A-2 750 395, US-A-2 863 881, US-A-3 187 018), sind solche Monomere und daraus formulierte kationisch polymerisierbare Massen mit den für dentale Anwendungen notwendigen Eigenschaften zu keinem Zeitpunkt kommerziell verfügbar gewesen.

Die Herstellung bifunktioneller cycloaliphatischer Epoxide wird bereits in Patenten der 50er Jahre beschrieben (US-A-2 750 395, US-A-900 506, US-A-907 149, US-A-2 745 847, US-A-2 853 499, US-A-3 187 018, US-A-2 863 881, US-A-2 853 498). Siliciumhaltige cycloaliphatische Epoxide wurden von Crivello et al. in verschiedenen Publikationen beschrieben (EP-A-0 449 027; J. Polym. Sci., Part A: Polym. Chem., 28 (1990) 479, ibid. 31 (1993) 2563; ibid. 31 (1993) 2729; ibid. 31 (1993) 3109; ibid. 31 (1993) 3121; ibid. 33 (1995) 2463). Es handelt sich bei den bekannten cycloaliphatischen Epoxiden im wesentlichen um niedermolekulare Monomere, die zwar einen etwas verminderten Polymerisationsschrumpf besitzen [J. Adhes. Sci. Technol. 9(10) 1995, 1343; DE-A-4 340 949], die aber aufgrund ihrer hohen Funktionsdichte den Anforderungen (Verarbeitung, physikalische Eigenschaften) für dentale Anwendungen nicht genügen.

Über kationisch härtbare Epoxidmassen für dentale Anwendungen ist nur wenig bekannt: Das Patent US-A-5 556 896 beschreibt epoxidhaltige Massen, die notwendigerweise als schrumpfkompensierende Monomere Spiroorthocarbonate enthalten müssen. Die Fa. Ciba beschrieb 1958 im Patent AT-A-204 687 Epoxid-Dentalmassen auf Basis von Bisphenol A, die mittels Lewis-Säure-Katalysatoren ausgehärtet wurden. Problematisch bei diesen Zubereitungen war die lange Aushärtzeit, bzw. die geringe mechanische Festigkeit und Langzeitstabilität. Die Fa. Minnesota Mining and Manufacturing Company und Wictorin et al. beschreiben in Patenten (WO 96/13538 und WO 95/30402) kationisch härtbare Epoxymischungen, vorzugsweise mit 3,4-Epoxycyclohexyl-methyl-3,4-epoxycyclohexancarboxylat bzw. Bis(3,4-epoxycylohexyladipat). Dieser Typ Epoxid ist in hohem Maße cytotoxisch und in-vitro Tests ergaben für diese Monomere mutagene Eigenschaften, die in der dentalen Anwendung unerwünscht sind.

Aufgabe der vorliegenden Erfindung ist es, polymerisierbare Massen bereitzustellen, die im Vergleich zu den bekannten Massen neben hoher Reaktivität und den notwendigen mechanischen Eigenschaften einen geringen Volumenschrumpf aufweisen und keine mutagenen und nur geringe cyctotoxische Eigenschaften besitzen.

Erfindungsgemäß wird diese Aufgabe gelöst durch polymerisierbare Massen, enthaltend
(a) 3 bis 80 Gew.-%, vorzugsweise 3 bis 75 Gew.-% und insbesondere 5 bis 70 Gew.-% eines Epoxids oder eines Gemisches von Epoxiden der allgemeinen Formel: 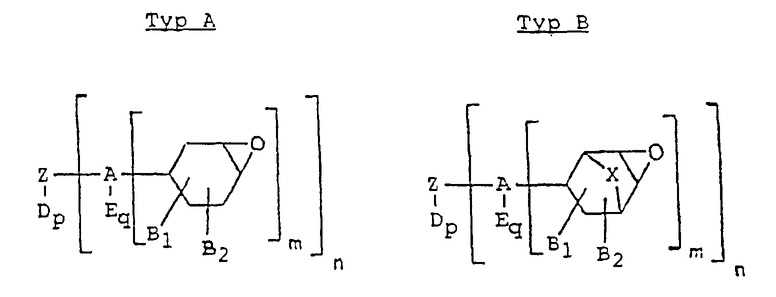 in welcher für Typ A bedeuten:
   bei n = 2
   - Z: einen cycloaliphatischen oder aromatischen Rest mit 1 bis 22, vorzugsweise 1 bis 18 C-Atomen oder eine Kombination dieser Reste, wobei ein oder mehrere C-Atome durch O, C=O, -O(C=O)-, SiR₂ und/oder NR ersetzt sein können, oder einen aliphatischen Rest mit 0 bis 22, vorzugsweise 1 bis 18 C-Atomen, wobei ein oder mehrere C-Atome durch 0, C=O, -O(C=O)-, NR oder SiR₂ ersetzt sein können, wobei im Falle einen aliphatischen Restes mindestens ein C-Atom durch SiR₂ ersetzt sein muß, und wobei R ein aliphatischer Rest mit 1 bis 7 C-Atomen ist, wobei ein oder mehrere C-Atome durch O, C=O und/oder -O(C=O)- ersetzt sein können,
   bei n > 2
   - Z: einen aliphatischen, cycloaliphatischen oder aromatischen Rest mit 0 bis 22, vorzugsweise 0 bis 18 C-Atomen oder eine Kombination dieser Reste, wobei ein oder mehrere C-Atome durch O, C=O, -O(C=O)-, SiR₂ und/oder NR ersetzt sein können und wobei R ein aliphatischer Rest mit 1 bis 7 C-Atomen ist, wobei ein oder mehrere C-Atome durch O, C=O und/oder -O(C=O)- ersetzt sein können,
   und in welcher für Typ B bedeuten:
   - Z: einen aliphatischen, cycloaliphatischen oder aromatischen Rest mit 0 bis 22, vorzugsweise 0 bis 18 C-Atomen oder eine Kombination dieser Reste, wobei ein oder mehrere C-Atome durch O, C=O, -O(C=O)-, SiR₂ und/oder NR ersetzt sein können und wobei R ein aliphatischer Rest mit 1 bis 7 C-Atomen ist, wobei ein oder mehrere C-Atome durch O, C=O und/oder -O(C=O)- ersetzt sein können,
   und in welcher für Typ A und Typ B bedeuten:
   - A: einen aliphatischen, cycloaliphatischen oder aromatischen Rest mit 1 bis 18, vorzugsweise 1 bis 15 C-Atomen oder eine Kombination dieser Reste, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atome durch O, C=O und/oder -O(C=O)- ersetzt sein können,
   - B₁, B₂, D, E: unabhängig voneinander ein H-Atom oder einen aliphatischen Rest mit 1 bis 9, vorzugsweise 1 bis 7 C-Atomen, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atome durch O, C=O und/oder -O(C=O)- ersetzt sein können,
   - n: 2-7, vorzugsweise 2-5, insbesondere 2-4,
   - m: 1-10, vorzgusweise 1-7, insbesondere 1-5,
   - p: 1-5, vorzugsweise 1-4, insbesondere 1 oder 2,
   - q: 1-5, vorzugsweise 1-4, insbesondere 1 oder 2 und
   - X: CH₂, S oder O
(b) 0 bis 80, vorzugsweise 0 bis 70 Gew.-% eines Epoxids oder eines Gemisches von Epoxiden, die von (a) verschieden sind,
(c) 3 bis 85, vorzugsweise 5 bis 75 Gew.-% Füllstoffe
(d) 0,01 bis 25, vorzugsweise 0,01 bis 20 Gew.-% Initiatoren, Verzögerer und/oder Beschleuniger,
(e) 0 bis 25, vorzugsweise 0 bis 20 Gew.-% Hilfsstoffe,
wobei die Prozentangaben jeweils auf das Gesamtgewicht der Masse bezogen sind.

Bevorzugte erfindungsgemäße Massen enthalten als Komponente
(a) eines oder mehrere der nachstehend aufgeführten Epoxide:
   i) 2,2-Bis[4,1-phenylenoxy-3,1-propandiyl-3,4-epoxycyclohexylcarbonsäureester]propyliden 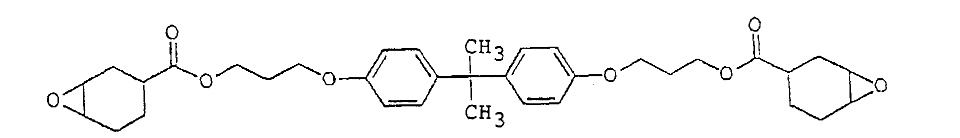
   ii) 2,2-Bis[4,1-phenylenoxy-3,1-propandiyl-oxy-methandiyl-3,4-epoxycyclohexyl]propyliden 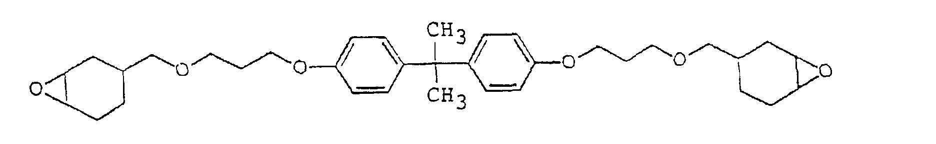
   iii) 2,2-Bis[3,4-epoxycyclohexylmethandiyl(4,1-phenylenoxy-3,1-propylcarbonsäureester)]propyliden 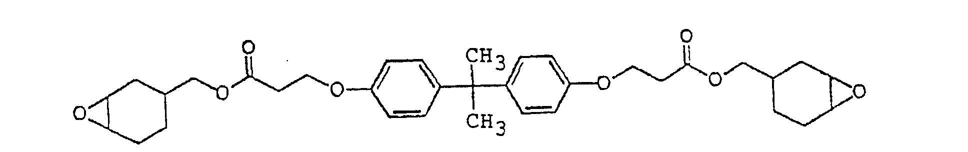
   iv) 2,2-Bis[4,1-phenylenoxy-3,1-propandiyl-1,1,3,3-tetramethyldisiloxanyl-1,2-ethandiyl-3,4-epoxycyclohexyl]-propyliden 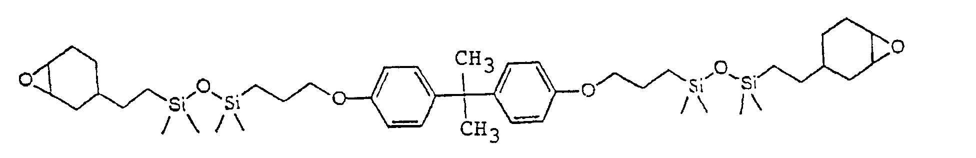
   v) 2,2-Bis{4,1-phenylenoxy-3,1-propandiyl-3-oxatricyclo-[3.2,1.0^{2,4}]octyl-6-carboxy}propyliden 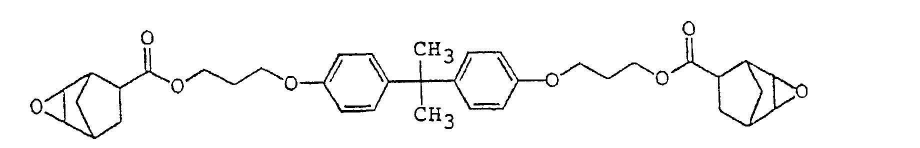
   vi) 2,2-Bis{4,1-phenylenoxy-3,1-propandiyl-3,8-dioxatricyclo[3.2.1.0^{2,4}]octyl-6-carboxy}propyliden 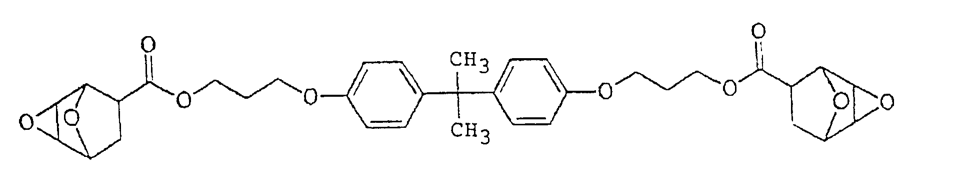
   vii) 2,2-Bis{4,1-phenylenoxy-3,1-propandiyl-[3,5,7-tris-(ethandiyl-3,4-epoxycyclohexyl)-1,3,5,7-tetramethylcyclotetrasiloxanyl]}propyliden 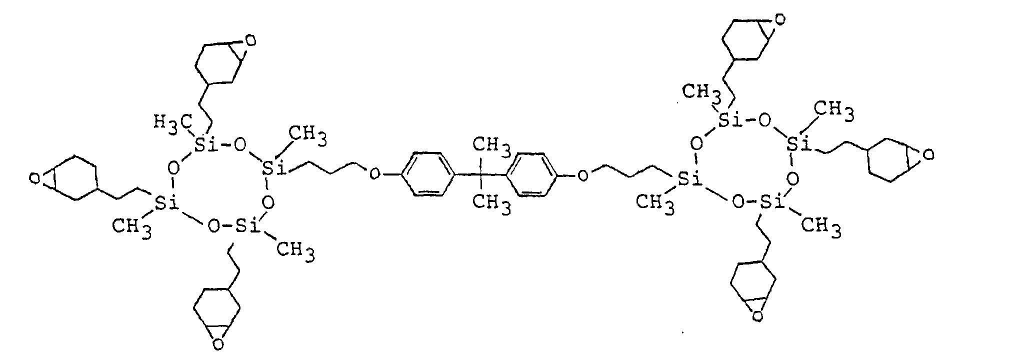
   viii) Bis[methandiyl-oxy-3,1-propandiyl-3,4-epoxycyclohexylcarbonsäureester]tricyclo[5.2.1.0^{2,6}]decan 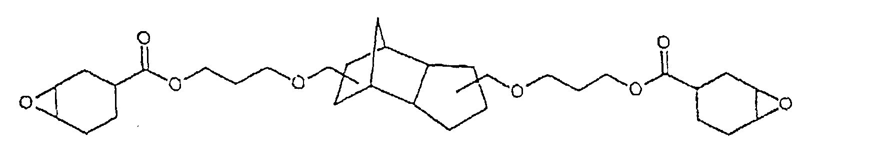
   ix) Bis[methandiyl-oxy-3,1-propandiyl-oxy-methandiyl-3,4-epoxycyclohexyl]tricyclo[5.2.1.0^{2,6}]decan 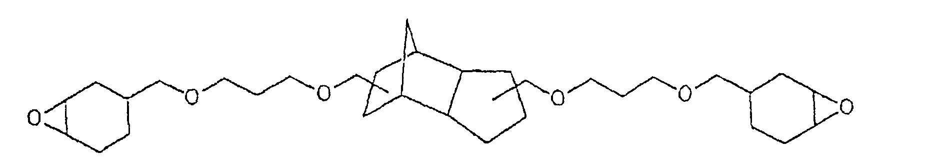
   x) Bis[3,4-epoxycyclohexylmethandiyl-propancarbonsäure-1-oxy-methandiyl]tricyclo[5.2.1.0^{2,6}]decan 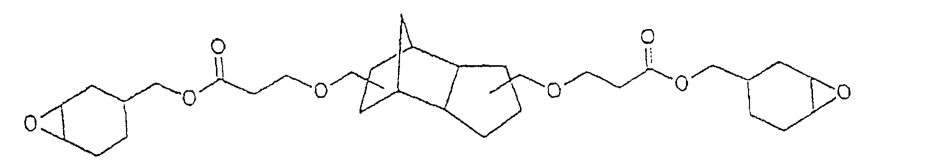
   xi) Bis(methandiyl-oxy-3,1-propandiyl-1,1,3,3-tetramethyldisiloxandiyl-1,2-ethandiyl-3,4-epoxycyclohexyl]tricyclo[5.2.1.0^{2,6}]decan 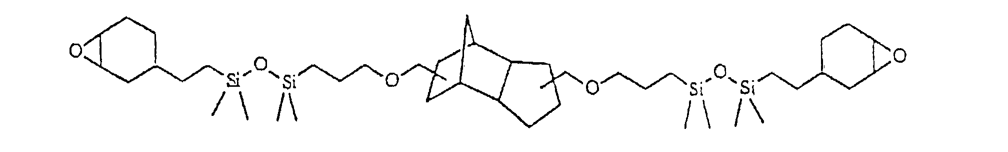
   xii) Bis{methandiyl-oxy-3,1-propandiyl-3-oxatricyclo-[3.2.1.0^{2,6}]octyl-6-carboxyl}tricyclo[5.2.1.0^{2,6}]-decan 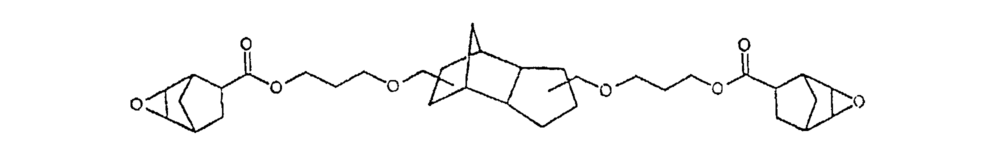
   xiii) Bis{methandiyl-oxy-3,1-propandiyl-3,8-dioxatricyclo-[3.2.1.0^{2,6}]octyl-6-carboxyl}tricyclo[5.2.1.0^{2,6}]-decan 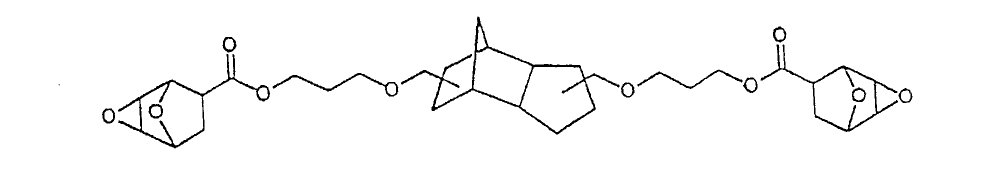
   xiv) Bis(methandiyl-oxy-(3-propandiyl-3,5,7-tris(2,1-ethandiyl-3,4-epoxycyclohexyl)-1,3,5,7-tetramethyl-cyclotetrasiloxanyl)-tricyclo[5.2.1.0^{2,6}]decan 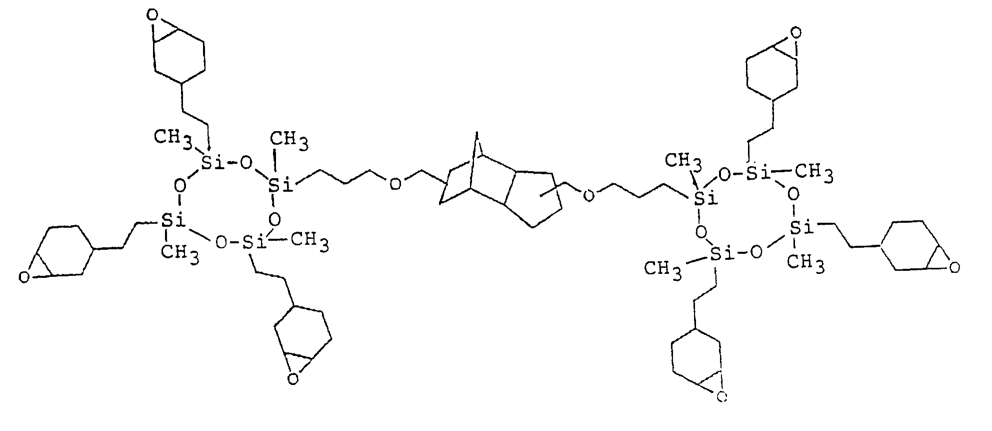
   xv) 1,1,1-Tris[methandiyl-oxy-methandiyl-3,4-epoxycyclohexyl]propan 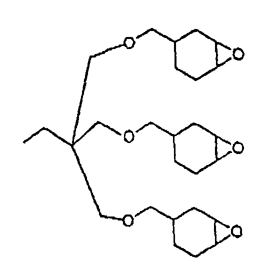
   xvi) 1,1,1-Tris[methandiyl-oxy-1,3-propandiyl-1,1,3,3-tetramethyldisiloxandiyl-1,2-ethandiyl-3,4-epoxy cyclohexyl]propan 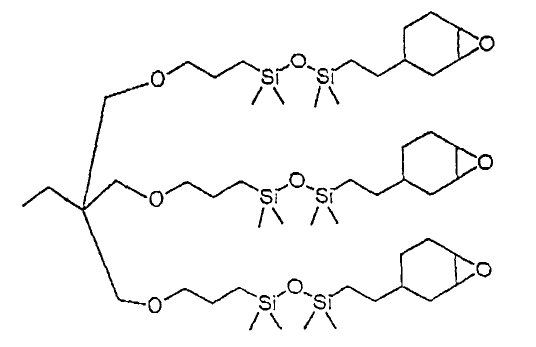
   xvii) 1,1,1-Tris{methandiyl-3-oxatricyclo[3.2.1.0^{2,4}]octyl-6-carboxy}propan 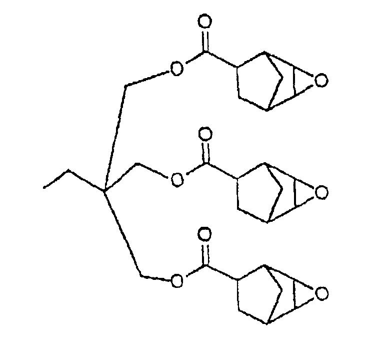
   xviii) 1,1,1-Tris{methandiyl-3,8-dioxatricyclo[3.2.1.0^{2,4}]-octyl-6-carboxy}propan 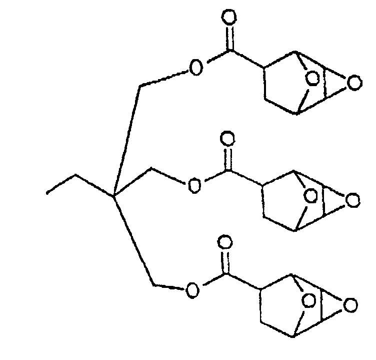
   xix) 1,1,1-Tris[methandiyl-oxy-3,1-propandiyl-3,5,7-tris-(2,1-ethandiyl-3,4-epoxycyclohexyl)-1,3,5,7-tetramethylcyclotetrasiloxanyl]propan 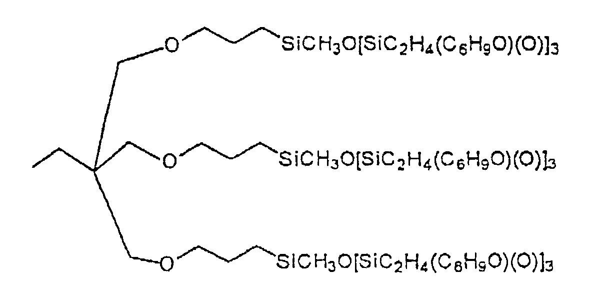
   xx) 1,1,1-Tris[methandiyl-oxy-bis(ethandiyloxy)-3,4-epoxycyclohexylcarbonsäureester]propan 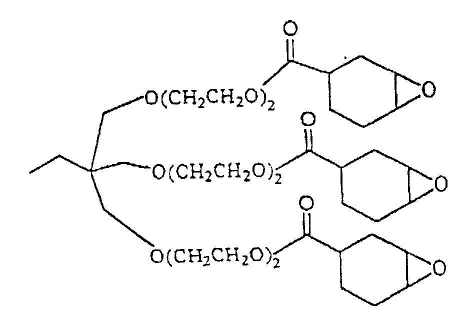
   xxi) 1,1,1-Tris[methandiyl-oxy-bis(ethandiyloxy)-methandiyl-3,4-epoxycyclohexyl]propan 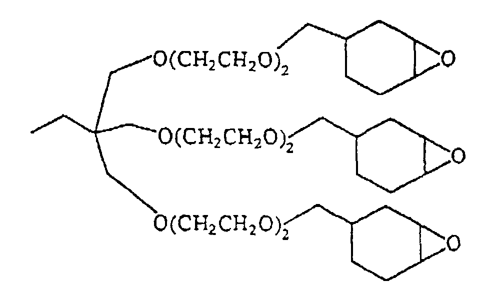
   xxii) 1,1,1-Tris[methandiyl-oxy-bis(ethandiyloxy)-propandiyl-1,1,3,3-tetramethyldisiloxanyl-1,2-ethandiyl-3,4-epoxycyclohexyl]propan 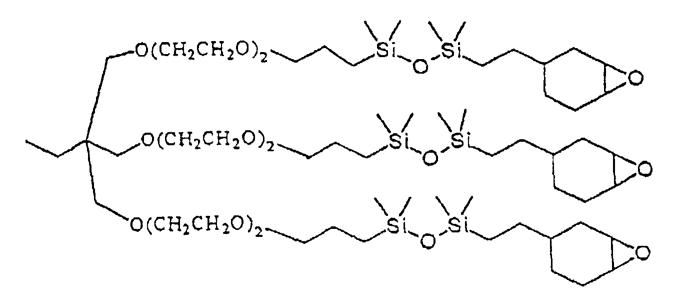
   xxiii) 1,1,1-Tris{methandiyl-oxy-bis(ethandiyloxy)-3-oxatricyclo[3.2.1.0^{2,4}]octyl-6-carboxy}propan 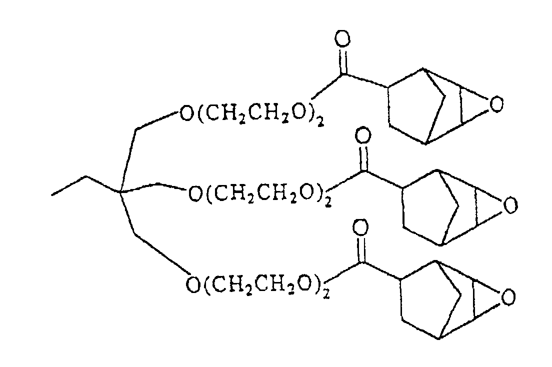
   xxiv) 1,1,1-Tris{methandiyl-oxy-bis(ethandiyloxy)-3,8-dioxatricyclo[3.2.1.0^{2,4}]octyl-6-carboxy}propan 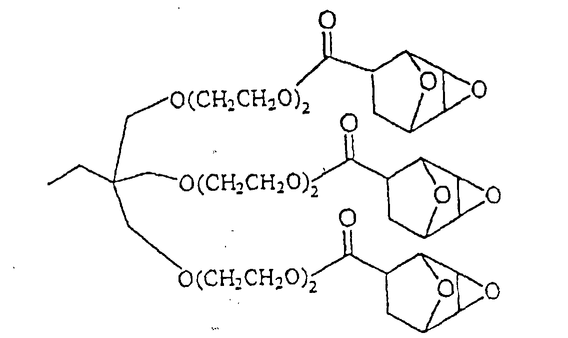
   xxv) 1,1,1-Tris[methandiyl-oxy-bis(ethandiyloxy)-propandiyl-3,5,7-tris(2,1-ethandiyl-3,4-epoxycyclohexyl)-1,3,5,7-tetramethylcyclotetrasiloxanyl]propan 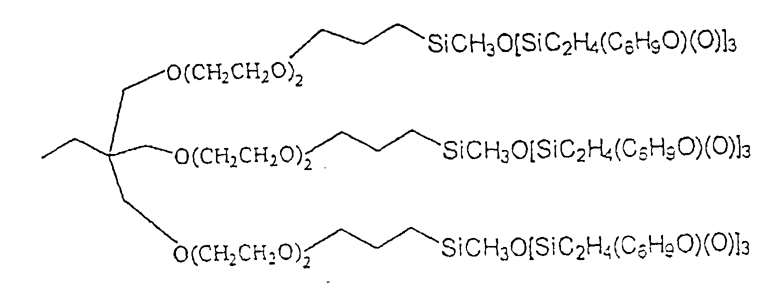
   xxvi) α,ω-Bis[3,4-epoxycyclohexylethandiyl-1,1,3,3-tetramethyldisiloxanyl-3,1-propandiyl]polytetrahydrofuran 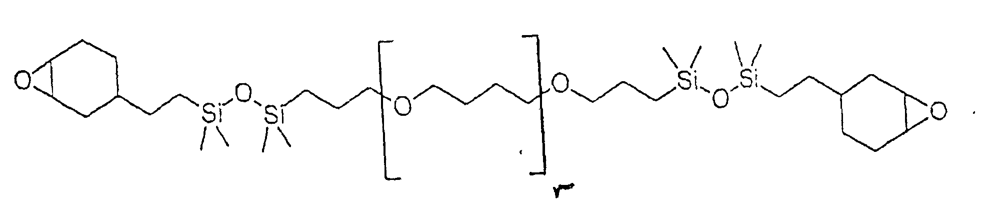
   xxvii) α,ω-Bis{3-oxatricyclo[3.2.1.0^{2,4}]octyl-6-carboxy}-polytetrahydrofuran 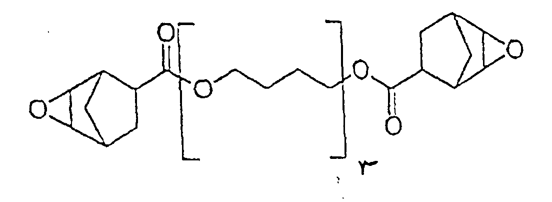
   xxviii) α,ω-Bis{3,8-dioxatricyclo[3.2.1.0^{2,4}]octyl-6-carboxy}polytetrahydrofuran 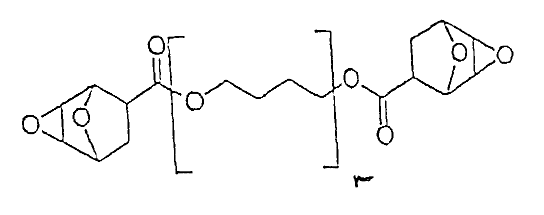
   xxix) α,ω-Bis-(3-propandiyl-3,5,7-tris(2,1-ethandiyl-3,4-epoxycyclohexyl)-1,3,5,7-tetramethylcyclotetrasiloxanyl)polytetrahydrofuran

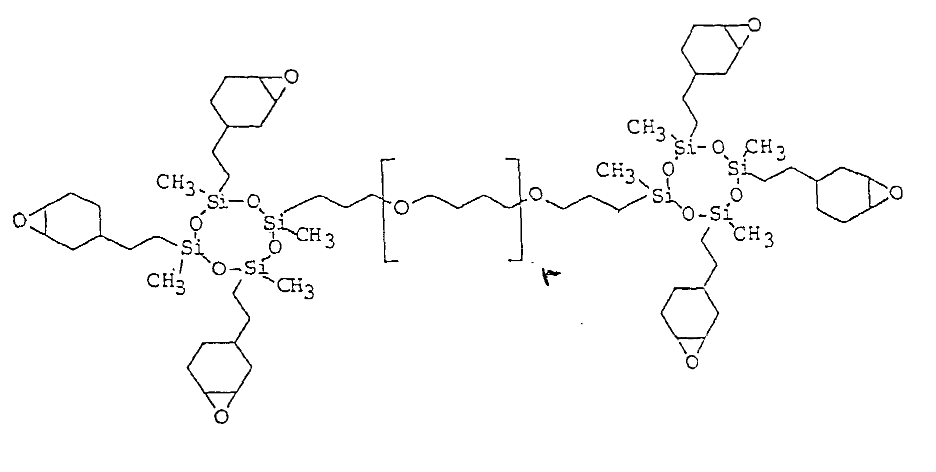

Die Erfindung bezieht sich auch auf die vorgenannten, einzeln aufgeführten neuen cycloaliphatischen Epoxide per se.

Die Epoxide können auf einfache Weise hergestellt werden. Zur Herstellung der Epoxide vom Typ A ohne Siloxanyl-Einheiten werden die durch klassische Veretherung bzw. Veresterung von Di-, Tri- oder Polyolen mit Cyclohexencarbonsäure bzw. Cyclohexenylmethanol erhaltenen cycloaliphatischen Alkene mit Perbenzoesäure im geeigneten Lösungsmittel, vorzugsweise in Diethylether, epoxidiert. Nach beendeter Reaktion wird mehrmals mit Natronlauge gewaschen und über Magnesiumsulfat getrocknet. Die flüchtigen Bestandteile werden im Vakuum abdestilliert.

Die Herstellung der Epoxide vom Typ A mit Siloxanyl-Einheiten gelingt durch zweistufige, klassische Hydrosilylierungen: Zu einem Überschuß eines Siloxans mit mindestens zwei aktiven Wasserstoffatomen und katalytischen Mengen H₂PtCl₆, gelöst in geeignetem Lösungsmittel (z.B. Hexan), werden Verbindungen mit zwei, drei oder mehreren terminalen Alken-Funktionen zugegeben. Nach beendeter Reaktion wird das Gemisch von ausgefallenem Platin befreit, einmal mit Wasser gewaschen und über Magnesiumsulfat getrocknet. In einer zweiten Hydrosilylierung wird die erhaltene di-, tri- oder polysiloxanhaltige Verbindung in einem geeigneten Lösungsmittel gelöst, z.B. in Toluol oder Alkanen, und mit H₂PtCl₆ und Vinylcyclohexenepoxid erhitzt. Das Gemisch wird von ausgefallenem Platin befreit und einmal mit Wasser gewaschen. Das Produkt erhält man, indem man die flüchtigen Bestandteile im Vakuum abdestilliert.

Zur Herstellung der Epoxide vom Typ B mit terminalen 3-Oxatricyclo[3.2.1.0^{2,4}]octanyl-Einheiten werden Di-, Tri- oder Poly(meth)acrylate mit substitutierten (oder unsubstituierten) monomerem Cyclopentadien, Thiophenen oder Furanen unter Lewis-Säure-Katalyse (beispielsweise ZnCl₂ oder AlCl₃) unter Normaldruck oder im Autoklaven zu den entsprechenden Diels-Alder-Produkten umgesetzt. Die Epoxidierung der endocyclischen Doppelbindung gelingt mit Perbenzoesäure im geeigneten Lösungsmittel, vorzugsweise in Diethylether. Nach beendeter Reaktion wird mehrmals mit Natronlauge gewaschen und über Magnesiumsulfat getrocknet. Die flüchtigen Bestandteile werden im Vakuum abdestilliert.

Die erfindungsgemäßen polymerisierbaren Massen können neben den erfindungsgemäßen Epoxiden als Komponente (b) andere, niederviskose Epoxide enthalten. Niederviskose Epoxide gemäß (b) können beispielsweise sein: 3,4-Epoxycyclohexylmethyl-3,4-epoxycyclohexancarboxylat (US-A-2 716 123), 3,4-Epoxy-6-methylcyclohexyl-3,4-epoxy-6-methylcyclohexancarboxylat (US-A-2 716 123) oder verwandte Epoxide, Vinylcyclohexendiepoxid (US-A-2 948 688), Dicyclopentadiendioxid (US-A-2 985 667), Bis(3,4-epoxycyclohexylmethyl)adipat (US-A-2 750 395, US-A-2 863 881, US-A-3 187 018), 1,3,5,7-Tetrakis(2,1-ethandiyl-3,4-epoxycyclohexyl)-1,3,5,7-tetramethylcyclotetrasiloxan der folgenden Formel: 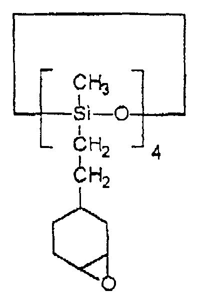 1,3,5,7,9-Pentakis(2,1-ethandiyl-3,4-epoxycyclohexyl)-1,3,5,7,9-pentamethylcyclopentasiloxan (US-A-5 085 124) der folgenden Formel: 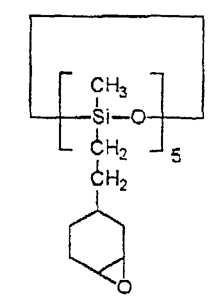 und niedermolekulare Siloxane, die mit cycloaliphatischen Epoxiden funktionalisiert sind, beispielsweise 1,1,3,3-Tetramethyl-1,3-bis(ethandiyl-3,4-epoxycyclohexyl)disiloxan (EP-A-0 449 027, EP-A-0 574 265) der folgenden Formel:

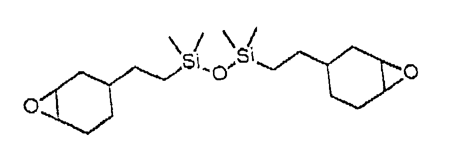

Die niederviskosen Epoxide gemäß Komponente (b) sind in einer Konzentration von 0 bis 80 Gew.-%, vorzugsweise 5 bis 75 Gew.-%, vorhanden, jeweils bezogen auf das Gesamtgewicht der Masse.

Anorganische Füllstoffe gemäß Komponente (c) können übliche dentale Füllstoffe, beispielsweise Quarz, gemahlene gegebenenfalls röntgenopake, gegebenenfalls reaktive Gläser, schwer lösliche Fluoride, wie CaF₂, YF₃ (EP-B-0 238 025), Kieselgele sowie pyrogene Kieselsäure oder deren Granulate sein. Ebenso können ein oder mehrere wasserlösliche anorganische komplexe Fluoride der allgemeinen Formel AₙMFₘ, worin A ein ein- oder mehrwertiges Kation, M ein Metall der III, IV oder V Haupt-oder Nebengruppe, n eine ganze Zahl von 1 bis 3 und m eine ganze Zahl von 4 bis 6 bedeuten (DE-A-4 445 266), als fluoridabgebende Bestandteile enthalten sein. Sie sind in einer Konzentration von 3 bis 85 Gew.-%, vorzugsweise 5 bis 75 Gew.-% und insbesondere 30 bis 75 Gew.-%, bezogen auf die Gesamtmasse, in den Dentalmassen enthalten. Zum besseren Einbau in die Polymermatrix kann es von Vorteil sein, die Füllstoffe sowie gegebenenfalls die röntgenopaken Zusatzstoffe, wie YF₃, zu hydrophobieren. Übliche Hydrophobierungsmittel sind Silane, beispielsweise Trimethoxyglycidylsilan. Die maximale Korngröße der anorganischen Füllstoffe beträgt vorzugsweise 20 µm, insbesondere 12 µm. Ganz besonders bevorzugt werden Füllstoffe mit einer mittleren Korngröße < 7 µm eingesetzt.

Initiatoren gemäß Komponente (d) der erfindungsgemäßen Massen können sein: Lewis- oder Broensted-Säuren bzw. Verbindungen, die solche Säuren freisetzen, welche die Polymerisation initiieren, beispielsweise BF₃ oder dessen etherische Addukte (BF₃·THF, BF₃·Et₂O, etc.), AlCl₃, FeCl₃, HPF₆, HAsF₆, HSbF₆, HBF₄ oder Substanzen, die nach Bestrahlen durch UV oder sichtbares Licht oder durch Wärme und/oder Druck die Polymerisation auslösen, wie z.B. (eta-6-Cu-mol)(eta-5-cyclopentadienyl)eisen-hexafluorophosphat, (eta-6-Cumol)(eta-5-cyclopentadienyl)eisen-tetrafluorobroat, (eta-6-Cumol)(eta-5-cyclopentadienyl)eisen-hexafluoroantimonat, substituierte Diaryliodoniumsalze und Triarylsulfoniumsalze. Als Beschleuniger können Peroxyverbindungen vom Typ der Perester, der Diacylperoxide, der Peroxydicarbonate und der Hydroperoxide eingesetzt werden. Bevorzugt werden Hydroperoxide verwendet, als besonders bevorzugter Beschleuniger kommt Cumolhydroperoxid in etwa 70 bis 90 %iger Lösung in Cumol zum Einsatz. Das Verhältnis von Photoinitiator zu Cumolhydroperoxid kann in weiten Grenzen von 1:0,001 bis 1:10 variiert werden, vorzugsweise wird jedoch ein Verhältnis von 1:0,1 bis 1:6 und besonders bevorzugt von 1:0,5 bis 1:4 verwendet. Die Verwendung von Komplexbildnern, wie beispielsweise Oxalsäure, 8-Hydroxychinolin, Ethylendiamintetraessigsäure und aromatischen Polyhydroxyverbindungen ist ebenfalls möglich. Als Verzögerer können Basen, typischerweise tertiäre Amine, zugesetzt werden. Die Komponente (d) liegt in der erfindungsgemäßen Masse in einer Menge von 0,01 bis 25 Gew.-%, vorzugsweise 0,01 bis 20 Gew.-%, bezogen auf das Gesamtgewicht der Masse, vor.

Geeignete Hilfsstoffe gemäß Komponente (e) können beispielsweise üblicherweise auf dem Dentalgebiet eingesetzte Stabilisatoren, Pigmente oder Verdünnungsmittel sein.

Die erfindungsgemäßen epoxidhaltigen, polymerisierbaren Massen eignen sich insbesondere als Werkstoffe für dentale Zwecke, beispielsweise zur Herstellung von Kunststoffzähnen oder Provisorien, als Beschichtungsmittel, zum Verkleben von Substraten sowie als dentale Füllungsmaterialien.

Einen besonderen Vorteil bieten die erfindungsgemäßen polymerisierbaren Massen bei dentalen Anwendungen. Der Volumenschrumpf der erfindungsgemäßen Massen liegt weit unterhalb des Schrumpfes der bekannten Massen, die auf Methacrylatmonomeren basieren. Dadurch kann beispielsweise die Randspaltproblematik bei Füllungsmaterialien weitgehend vermieden werden. Ebenso ist die Dimensionsstabilität und die Lagerstabilität der erfindungsgemäßen Epoxide und der daraus hergestellten polymerisierbaren Massen bei Präzisions-Modellmaterialien von großem Vorteil.

Gegenüber Dentalmassen auf Acrylatbasis, die im Vergleich zu epoxidbasierenden Massen eine sehr kurze Abbindungszeit und daher eine schlagartige Aushärtung aufweisen, zeigen die erfindungsgemäßen Massen eine über einen Zeitraum von beispielsweise 10 bis 240 Sekunden gleichmäßig ablaufende Erhärtung. Spannungen innerhalb der entstandenen Polymeren werden somit vermieden. Die erfindungsgemäßen Massen weisen daher auch eine optimale Verarbeitungszeit auf, bevor sie ihre endgültige Härte erreichen.

Die erfindungsgemäßen polymerisierbaren Massen zeigen neben hohen Druck- und Biegefestigkeiten überraschend hohe Schlagzähigkeiten, was für dentale Anwendungen herausragende Bedeutung besitzt.

Die erfindungsgemäßen dentalen Füllungsmaterialien zeigen außerdem eine überraschend gute Haftung an der Zahnsubstanz. Weiterhin sind die erfindungsgemäßen Epoxide nicht mutagen und hinsichtlich ihrer Cytotoxizität unbedenklich.

### Beispiele zur Synthese der erfindungsgemäßen Monomere

### 1. Herstellung von 2,2-Bis[4,1-phenylenoxy-3,1-propandiyl-3,4-epoxycyclohexylcarbonsäureester]propyliden:

100 g 2,2-Bis(4-oxypropylhydroxyphenyl)propan werden mit 85 g 4-Cyclohexencarbonsäure 6 Stunden in Toluol am Rückfluß gehalten. Das Lösungsmittel und die überschüssige 4-Cyclohexencarbonsäure werden abdestilliert. Zurück bleiben 161 g 2,2-Bis(4-oxypropylphenyl-3-cyclohexenylcarboxylat)propan, welche zu 83 g Perbenzoesäure in 500 ml Diethylether gegeben werden. Nach 8 Stunden Reaktionszeit wird mehrmals mit 10 %iger Natronlauge gewaschen und über Magnesiumsulfat getrocknet. Die flüchtigen Bestandteile werden im Vakuum abdestilliert.

### 2. Herstellung von Bis(3,4-epoxycyclohexylethyl-tetramethyldisiloxanylpropoxymethyl)-tricyclo[5.2.1.0^{2,6}]decan

174 g Tetramethyldisiloxan werden in 800 ml Hexan vorgelegt und mit 120 mg H₂PtCl₆ versetzt. Dazu werden 80 g Tricyclo[5.2.1.0^{2,6}]decan-diallylether gegeben und 3 Stunden auf 85°C (Sdp) erhitzt. Das Gemisch wird von ausgefallenem Platin befreit, einmal mit Wasser gewaschen und über Magnesiumsulfat getrocknet. Hexan und flüchtige Bestandteile werden im Vakuum abdestilliert. Das erhaltene Bis(tetramethyldisiloxanylpropoxymethyl)-tricyclo[5.2.1.0^{2,6}]decan wird in 200 ml Hexan gelöst mit 120 mg H₂PtCl₆ und 71 g Vinyl-3,4-cyclohexenepoxid versetzt und 3 Stunden auf 85°C erhitzt.

Das Gemisch wird von ausgefallenem Platin befreit, einmal mit Wasser gewaschen und über Magnesiumsulfat getrocknet. Man erhält nach Einengen der Reaktionsmischung im Vakuum 202 g Bis (3,4-epoxycyclohexylethyl-tetramethyldisiloxanylpropoxymethyl)-tricyclo[5.2.1.0^{2,6}]decan.

### 3. Herstellung von α,ω-Bis(3-propandiyl-3,5,7-tris(2,1-ethandiyl-3,4-epoxycyclohexyl)-1,3,5,7-tetramethylcyclotetrasiloxanyl)-polytetrahydrofuran

126 g α,ω-Polytetrahydrofuran-600-diallylether werden zu 151 g 1,3,5,7-Tetramethylcyclotetrasiloxan und 110 mg H₂PtCl₆ in 700 ml Hexan gegeben. Nach 3 Stunden bei 85°C wird ausgefallenes Platin abgetrennt und alle flüchtigen Bestandteile im Vakuum abdestilliert. Zurück bleibt α,ω-Bis-[3,5,7-tris(3,4-epoxycyclohexylethyl)-3-propyl-1,3,5,7-tetramethylcyclotetrasiloxanyl]-polytetrahydrofuran, welches mit 179 g Vinylcyclohexenepoxid und 130 mg H₂PtCl₆ in 800 ml Toluol 3 Stunden auf 120°C erhitzt wird. Ausgefallenes Platin wird abgetrennt und alle flüchtigen Bestandteile im Vakuum abdestilliert. Zurück bleiben 339 g α,ω-Bis-[3,5,7-tris(3,4-epoxycyclohexylethyl)-3-propyl-1,3,5,7-tetramethyl-cyclotetrasiloxanyl]-polytetrahydrofuran.

### 4. Herstellung von α,ω-Bis(6-methyl-dioxatricyclo-[3.2.1.0^{2,4}]octyl-6-carboxy)polyethylenglykol

85 g α,ω-Polyethylenglykol-600-dimethacrylat werden mit 19 g Furan in 300 ml Toluol im Autoklaven bei 10 bar 4 Stunden auf 120°C erhitzt. Die flüchtigen Bestandteile werden im Vakuum abdestilliert. Zurück bleibt α,ω-Bis(3-methyl-7-oxabicyclo[2.2.1]heptyl-3-carboxy)polyethylenglykol, das zu einer Lösung von 31 g Perbenzoesäure in 300 ml Diethylether zugetropft wird. Nach 8 Stunden Reaktionszeit wird mehrmals mit 10 %iger Natronlauge gewaschen und über Magnesiumsulfat getrocknet. Die flüchtigen Bestandteile werden im Vakuum abdestilliert.

### 5. Herstellung von 1,1,1-Tris[methandiyl-oxy-bis(ethandiyloxy)-methandiyl-3,4-epoxycyclohexyl]propan

168 g 1,1,1-Tris[methandiyl-hydroxy-bis(ethandiyloxy)]propan werden in 600 ml Tetrahydrofuran vorgelegt. Es werden portionsweise 178 g Kalium-tert.-butanolat zugegeben und eine Stunde bei 30°C gerührt. Anschließend werden 277 g 3-Cyclohexenylbrommethan, gelöst in 300 ml Tetrahydrofuran, zugetropft. Die Reaktionsmischung wird 24 Stunden auf 75°C erhitzt. Das Lösungsmittel wird abdestilliert, das Gemisch mit Methyl-tert.-butylether aufgeschlämmt und der Niederschlag abfiltriert. Nach wäßriger Extraktion verbleiben 249 g 1,1,1-Tris[methandiyl-oxy-bis(ethandiyloxy)-methandiyl-3-cyclohexenyl]propan, die in 500 ml Diethylether gelöst werden. Diese Lösung wird zu 61 g Perbenzoesäure in 400 ml Diethylether gegeben und nach 8-stündiger Reaktionszeit mehrmals mit 10 %iger Natronlauge gewaschen. Das Gemisch wird über Magnesiumsulfat getrocknet und die flüchtigen Bestandteile werden im Vakuum abdestilliert.

### Beispiele für polymerisierbare Massen

### 1. Allgemeine Herstellung polymerisierbarer Massen

### 1.1 Einkomponentige, licht- oder UV-härtende Systeme

Die Herstellung der erfindungsgemäß polymerisierbaren, einkomponentigen Massen ist ein zweistufiges Verfahren, bei dem in der ersten Stufe eine homogene Vormischung aller Bestandteile mit Ausnahme des Photoinitiators erfolgt. In einer zweiten Stufe wird der Photoinitiator unter Ausschluß von Licht homogen in die Paste eingeknetet. Nach vollständiger Einmischung der so erhaltenen verarbeitungsfähigen erfindungsgemäßen Epoxidharze erfolgt die Abfüllung in lichtundurchlässige Behälter.

### 1.2 Zweikomponentige Systeme

Die Herstellung der erfindungsgemäß polymerisierbaren zweikomponentigen Massen erfolgt, indem zunächst eine homogen gemischte Komponente A hergestellt wird, bestehend aus den Epoxid-Monomeren, einem Füllstoffanteil, den Verzögerern, Beschleunigern und den Hilfsstoffen. Weiterhin wird eine homogen gemischte Komponente B hergestellt, bestehend aus dem Initiator, Verdünnungsmittel und einem weiteren Füllstoffanteil. Die Komponenten A und B werden beispielsweise in ein Doppelkartuschensystem eingefüllt. Durch einen statischen Mischer am Kartuschensystem ist die zweikomponentige Masse direkt verarbeitbar.

### 2. Anwendungsbeispiele

### Beispiel 1

Komponente A: Aus 17,6 Gew.-Teilen α,ω-Bis-(3-propandiyl-3,5,7-tris(2,1-ethandiyl-3,4-epoxycyclohexyl)-1,3,5,7-tetramethylcyclotetrasiloxanyl)-ditetrahydrofuran, 17,6 Gew.-Teilen 3,4-Epoxycyclohexylmethyl-3,4-epoxycyclohexancarboxylat und 64,7 Gew.-Teilen eines silanisierten, pigmentierten Quarzes wird eine Paste geknetet. Eine zweite Komponente B besteht aus 32,8 Gew.-Teilen Polyethylenglykol, 4,7 Gew.-Teilen BF₃·OEt₂ und 62,5 Gew.-Teilen eines silanisierten, pigmentierten Quarzes. Die Pasten A und B werden in einem Verhältnis 2:1 über einen statischen Mischer gemischt. Die Masse härtet innerhalb von zwei Minuten aus.

### Beispiel 2

Aus 18 Gew.-Teilen Bis(methandiyl-oxy-3,1-propandiyl-3,4-epoxycyclohexylcarbonsäureester)-tricyclo-[5.2.1.0^{2,6}]decan, 10 Gew.-Teilen 1,1,3,3-Tetramethyl-1,3-bis(ethandiyl-3,4-epoxycyclohexyl)disiloxan, 68,9 Gew.-Teilen eines silanisierten, pigmentierten Quarzes, 1,2 Gew.-Teilen (eta-6-Cumol)(eta-5-cyclopentadienyl)-eisenhexafluorophosphat und 1,8 Gew.-Teilen Cumolhydroperoxid wird eine Paste geknetet, die durch Bestrahlen mit einer Lampe (Lichtgerät Elipar II, ESPE Dental-Medizin GmbH & Co. KG, Deutschland) in ca. 40 Sekunden ausgehärtet wird.

### Beispiel 3

18 Gew.-Teile 2,2-Bis[4,1-phenylenoxy-3,1-propandiyl-3,4-epoxycyclohexylcarbonsäureester]propyliden, 13 Gew.-Teile 3,4-Epoxycyclohexylmethyl-3,4-epoxycyclohexancarboxylat, 65,4 Gew.-Teile eines silanisierten pigmentierten Quarz, 1,5 Gew.-Teile (eta-6-Cumol)(eta-5-cyclopentadienyl)eisen-hexafluorophosphat und 2,1 Gew.-Teile Cumolhydroperoxid werden zu einer Paste geknetet, die durch Bestrahlen mit einer Lampe (Lichtgerät Elipar II, ESPE Dental-Medizin GmbH & Co. KG, Deutschland) in ca. 40 Sekunden ausgehärtet wird.

### Beispiel 4

| Vergleichspaste, käufliche Dentalmasse | | | | |
|---|---|---|---|---|
| | Beispiel 1 | Beispiel 2 | Beispiel 3 | Beispiel 4 (Vergleich) |
| Druckfestigkeit [MPa] | 388 | 331 | 355 | 385 |
| Biegefestigkeit [MPa] (ISO 4049) | 151 | 131 | 141 | 118 |
| Volumenschrumpf [%] | 1.3 | 0,8 | 1,0 | 2,2 |

## Patentansprüche

1. Polymerisierbare Masse, enthaltend
(a) 3 bis 80 Gew.-% eines Epoxids oder eines Gemisches von Epoxiden der allgemeinen Formel: 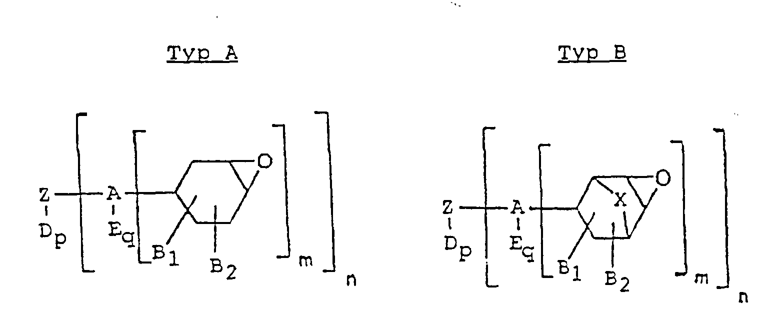 in welcher für Typ A bedeuten:
bei n = 2
Z einen cycloaliphatischen oder aromatischen Rest mit 1 bis 22 C-Atomen oder eine Kombination dieser Reste, wobei ein oder mehrere C-Atome durch O, C=O, -O(C=O)-, SiR₂ und/oder NR ersetzt sein können, oder einen aliphatischen Rest mit 0 bis 22 C-Atomen, wobei ein oder mehrere C-Atome durch O, C=O, -O(C=O)-, NR oder SiR₂ ersetzt sein können, wobei im Falle einen aliphatischen Restes mindestens ein C-Atom durch SiR₂ ersetzt sein muß, und wobei R ein aliphatischer Rest mit 1 bis 7 C-Atomen ist, wobei ein oder mehrere C-Atome durch O, C=O und/oder -O(C=O)- ersetzt sein können,
bei n > 2
Z einen aliphatischen, cycloaliphatischen oder aromatischen Rest mit 0 bis 22 C-Atomen oder eine Kombination dieser Reste, wobei ein oder mehrere C-Atome durch O, C=O, -O(C=O)-, SiR₂ und/oder NR ersetzt sein können und wobei R ein aliphatischer Rest mit 1 bis, 7 C-Atomen ist, wobei ein oder mehrere C-Atome durch 0, C=O und/oder -O(C=O)- ersetzt sein können,
und in welcher für Typ B bedeuten:
Z einen aliphatischen, cycloaliphatischen oder aromatischen Rest mit 0 bis 22 C-Atomen oder eine Kombination dieser Reste, wobei ein oder mehrere C-Atome durch O, C=O, -O(C=O)-, SiR₂ und/oder NR ersetzt sein können und wobei R ein aliphatischer Rest mit 1 bis 7 C-Atomen ist, wobei ein oder mehrere C-Atome durch O, C=O und/oder -O(C=O)- ersetzt sein können,
und in welcher für Typ A und Typ B bedeuten:
A einen aliphatischen, cycloaliphatischen oder aromatischen Rest mit 1 bis 18 C-Atomen oder eine Kombination dieser Reste, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atome durch O, C=O und/oder -O(C=O)- ersetzt sein können,
B₁, B₂, D, E unabhängig voneinander ein H-Atom oder einen aliphatischen Rest mit 1 bis 9 C-Atomen, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atone durch O, C=O und/oder -O(C=O)- ersetzt sein können,
n 2-7,
m 1-10,
p 1-5,
q 1-5 und
X CH₂, S oder O,
(b) 0 bis 80 Gew.-% eines Epoxids oder eines Gemisches von Epoxiden, die von (a) verschieden sind,
(c) 3 bis 85 Gew.-% Füllstoffe
(d) 0,01 bis 25 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Masse bezogen sind.

2. Polymerisierbare Masse nach Patentanspruch 1, **dadurch gekennzeichnet, daß** sie als Komponente (a) eines oder mehrere der folgenden Epoxide enthält:
i) 2,2-Bis[4,1-phenylenoxy-3,1-propandiyl-3,4-epoxycyclohexylcarbonsäureester]propyliden 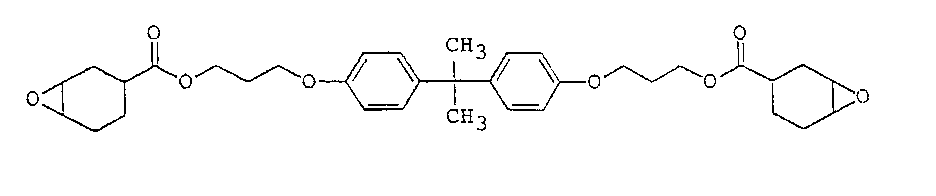
ii) 2,2-Bis[4,1-phenylenoxy-3,1-propandiyl-oxymethandiyl-3,4-epoxycyclohexyl]propyliden 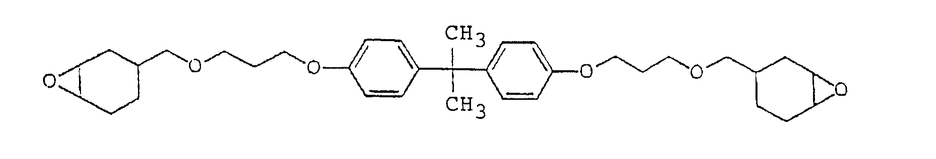
iii) 2,2-Bis[3,4-epoxycyclohexylmethandiyl(4,1-phenylenoxy-3,1-propylcarbonsäureester)]propyliden 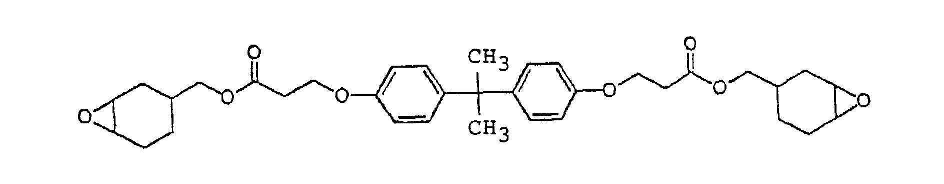
iv) 2,2-Bis[4,1-phenylenoxy-3,1-propandiyl-1,1,3,3-tetramethyldisiloxanyl-1,2-ethandiyl-3,4-epoxycyclohexyl]propyliden 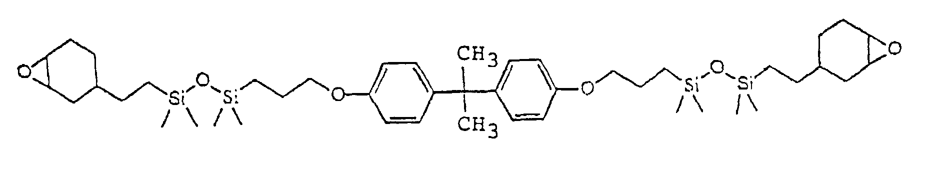
v) 2,2-Bis{4,1-phenylenoxy-3,1-propandiyl-3-oxatricyclo[3.2.1.0^{2,4}]octyl-6-carboxy}propyliden 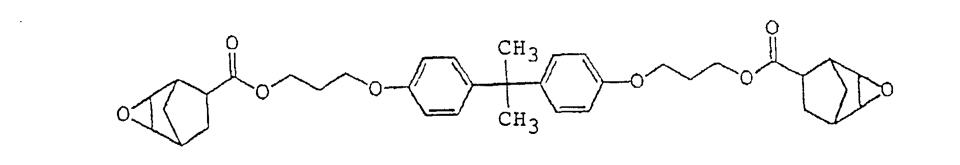
vi) 2,2-Bis{4,1-phenylenoxy-3,1-propandiyl-3,8-dioxatricyclo[3.2.1.0^{2,4}]octyl-6-carboxy}-propyliden 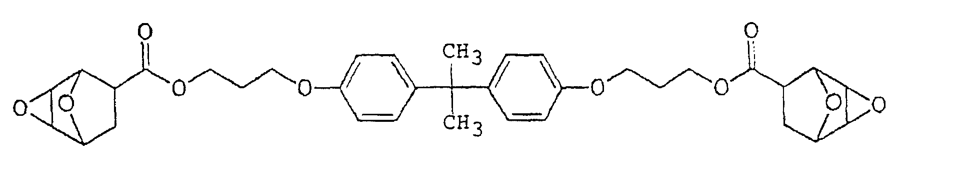
vii) 2,2-Bis{4,1-phenylenoxy-3,1-propandiyl-[3,5,7-tris(ethandiyl-3,4-epoxycyclohexyl)-1,3,5,7-tetramethylcyclotetrasiloxanyl]}propyliden 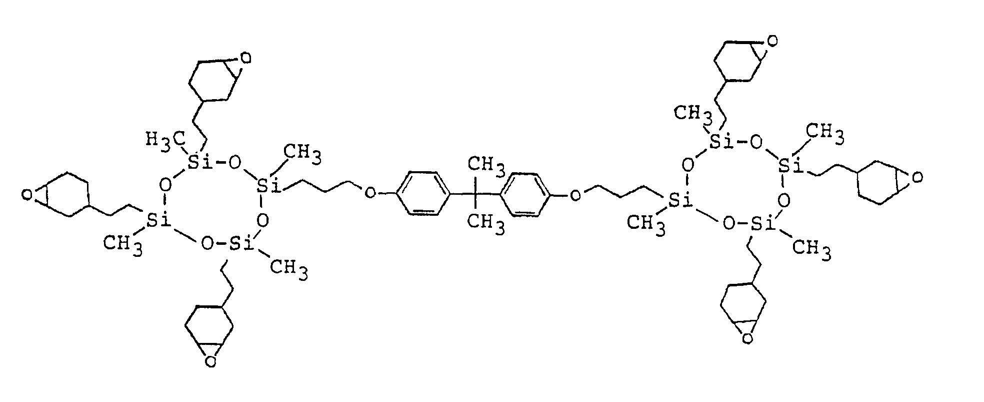
viii) Bis[methandiyl-oxy-3,1-propandiyl-3,4-epoxycyclohexylcarbonsäureester]tricyclo[5.2.1.0^{2,6}]-decan 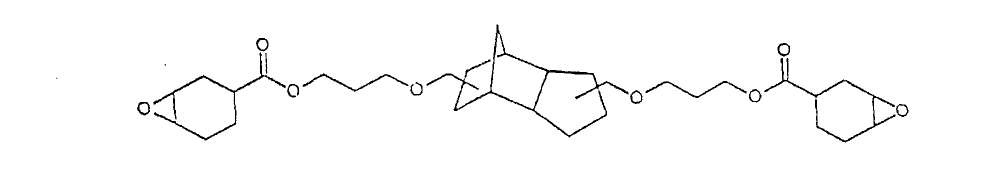
ix) Bis[methandiyl-oxy-3,1-propandiyl-oxy-methandiyl-3,4-epoxycyclohexyl]tricyclo[5.2.1.0^{2,6}]-decan 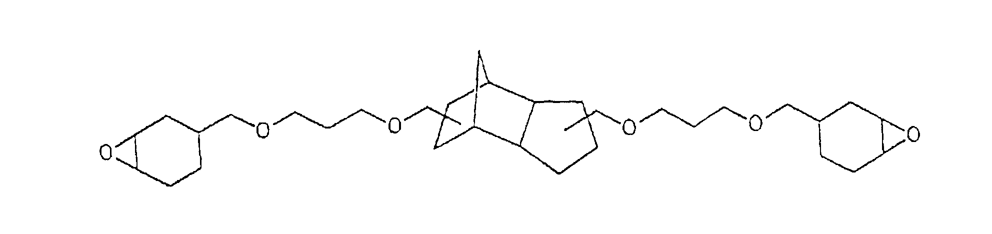
x) Bis[3,4-epoxycyclohexylmethandiyl-propancarbonsäure-1-oxy-methandiyl]tricyclo[5.2.1.0^{2,6}]-decan 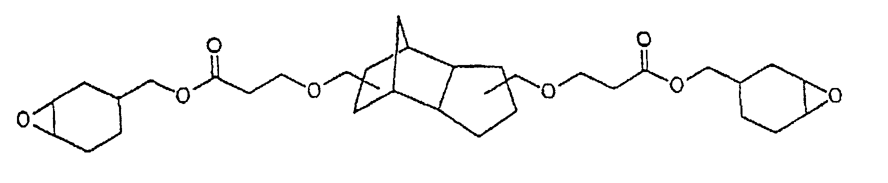
xi) Bis(methandiyl-oxy-3,1-propandiyl-1,1,3,3-tetramethyldisiloxandiyl-1,2-ethandiyl-3,4-epoxycyclohexyl]tricyclo[5.2.1.0^{2,6}]decan 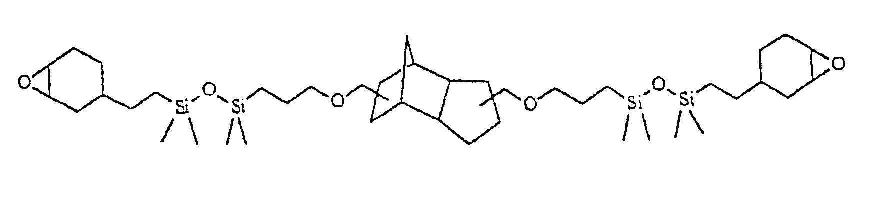
xii) Bis{methandiyl-oxy-3,1-propandiyl-3-oxatricyclo[3.2.1.0^{2,6}]octyl-6-carboxyl}tricyclo-[5.2.1.0^{2,6}]decan 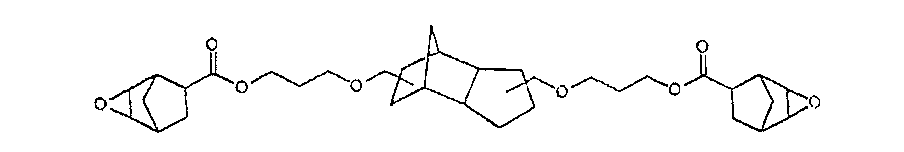
xiii) Bis{methandiyl-oxy-3,1-propandiyl-3,8-dioxatricyclo[3.2.1.0^{2,6}]octyl-6-carboxyl}tricyclo-[5.2.1.0^{2,6}]decan 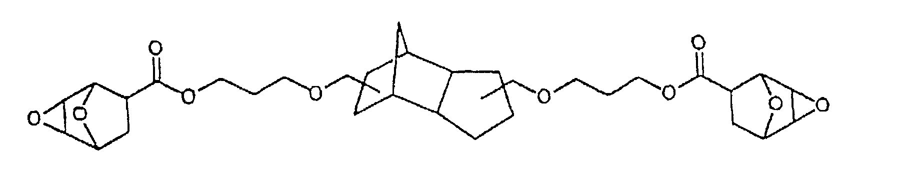
xiv) Bis(methandiyl-oxy-(3-propandiyl-3,5,7-tris(2,1-ethandiyl-3,4-epoxycyclohexyl)-1,3,5,7-tetramethylcyclotetrasiloxanyl)-tricyclo[5.2.1.0^{2,6}]-decan 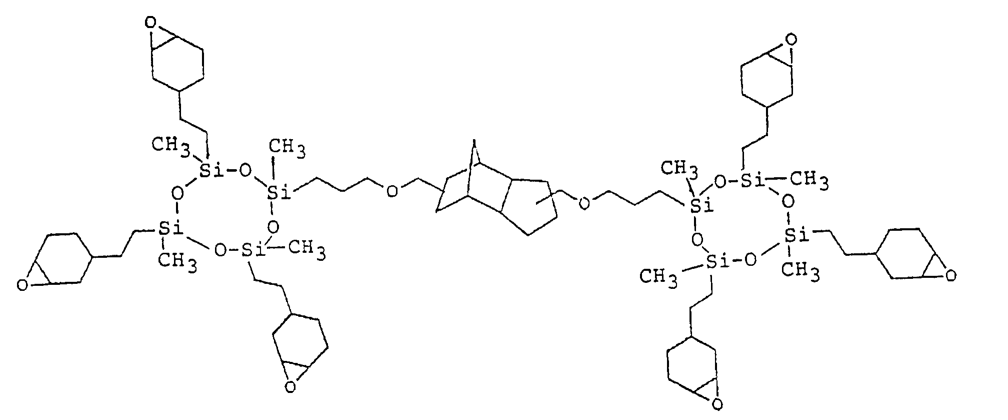
xv) 1,1,1-Tris[methandiyl-oxy-methandiyl-3,4-epoxycyclohexyl]propan 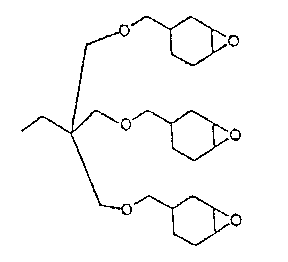
xvi) 1,1,1-Tris[methandiyl-oxy-1,3-propandiyl-1,1,3,3-tetramethyldisolxandiyl-1,2-ethandiyl-3,4-epoxycyclohexyl]propan 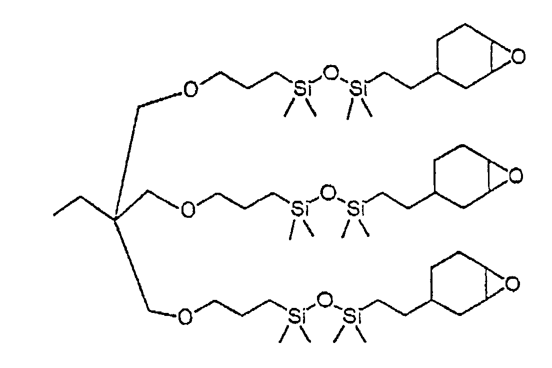
xvii) 1,1,1-Tris{methandiyl-3-oxatricyclo-[3.2.1.0^{2,4}]octyl-6-carboxy}propan 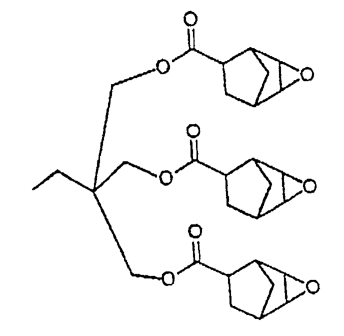
xviii) 1,1,1-Tris{methandiyl-3,8-dioxatricyclo-[3.2.1.0^{2,4}]octyl-6-carboxy}propan 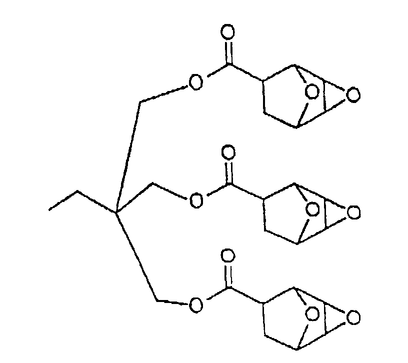
xix) 1,1,1-Tris[methandiyl-oxy-3,1-propandiyl-3,5,7-tris(2,1-ethandiyl-3,4-epoxycyclohexyl)-1,3,5,7-tetramethylcyclotetrasiloxanyl]propan 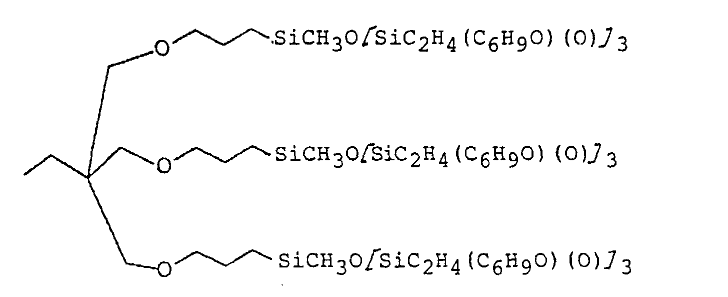
xx) 1,1,1-Tris[methandiyl-oxy-bis(ethandiyloxy)-3,4-epoxycyclohexylcarbonsäureester]propan 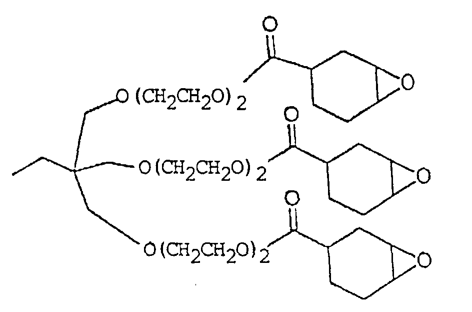
xxi) 1,1,1-Tris[methandiyl-oxy-bis(ethandiyloxy)-methandiyl-3,4-epoxycyclohexyl]propan 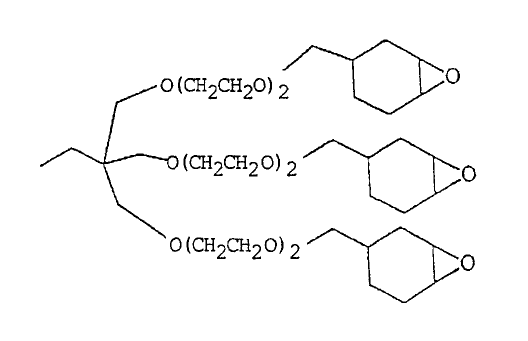
xxii) 1,1,1-Tris[methandiyl-oxy-bis(ethandiyloxy)-propandiyl-1,1,3,3-tetramethyldisiloxanyl-1,2-ethandiyl-3,4-epoxycyclohexyl]propan 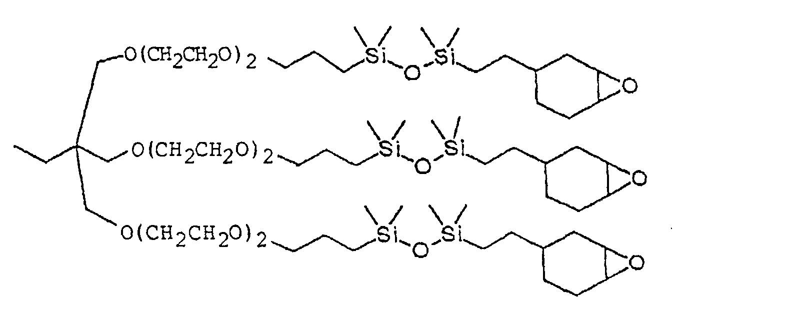
xxiii) 1,1,1-Tris{methandiyl-oxy-bis(ethandiyloxy)-3-oxatricyclo[3.2.1.0^{2,4}]octyl-6-carboxy}propan 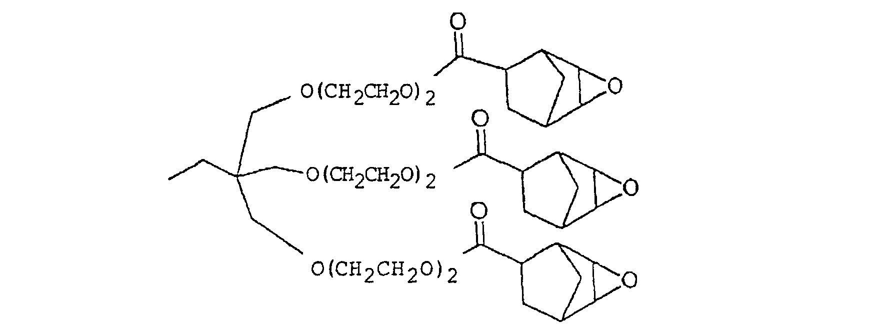
xxiv) 1,1,1-Tris{methandiyl-oxy-bis(ethandiyloxy)-3,8-dioxatricyclo[3.2.1.0^{2,4}]octyl-6-carboxy}propan 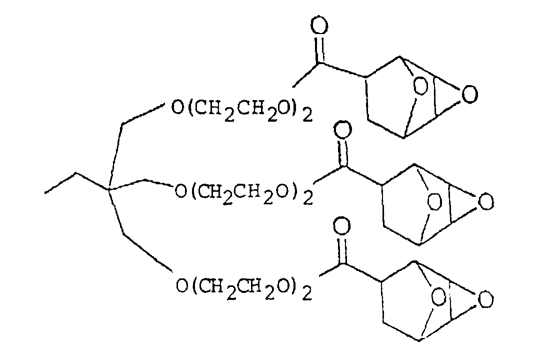
xxv) 1,1,1-Tris[methandiyl-oxy-bis(ethandiyloxy)-propandiyl-3,5,7-tris(2,1-ethandiyl-3,4-epoxycyclohexyl)-1,3,5,7-tetramethylcyclotetrasiloxa nyl]propan 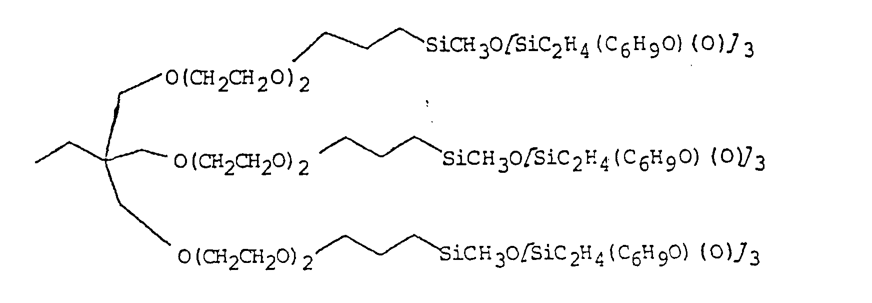
xxvi) α,ω-Bis[3,4-epoxycyclohexylethandiyl-1,1,3,3-tetramethyldisiloxanyl-3,1-propandiyl]polytetrahydrofuran 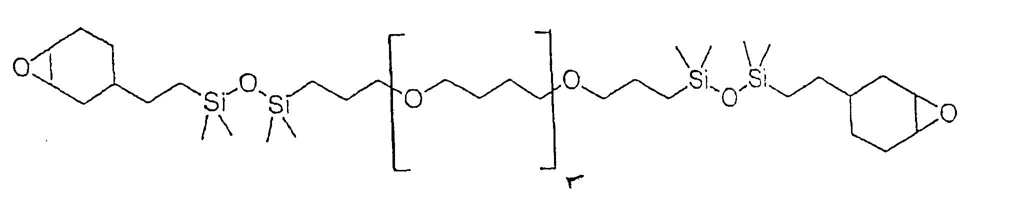
xxvii) α,ω-Bis{3-oxatricyclo[3.2.1.0^{2,4}]octyl-6-carboxy}polytetrahydrofuran 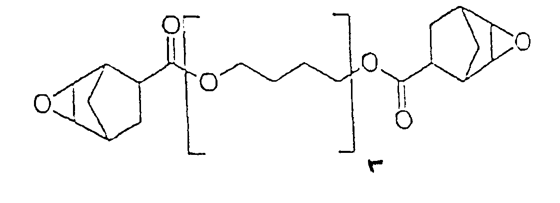
xxviii) α,ω-Bis{3,8-dioxatricyclo[3.2.1.0^{2,4}]octyl-6-carboxy}polytetrahydrofuran 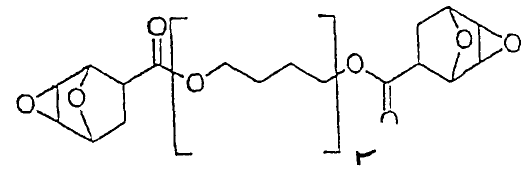
xxix) α,ω-Bis-(3-propandiyl-3,5,7-tris(2,1-ethandiyl-3,4-epoxycyclohexyl)-1,3,5,7-tetramethylcyclotetrasiloxanyl)polytetrahydrofuran

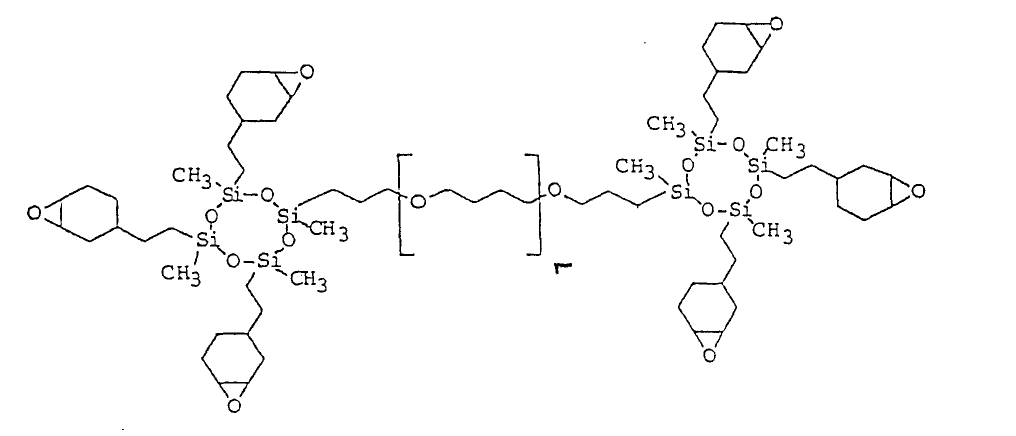

3. Polymerisierbare Masse nach Patentanspruch 1 oder 2, **dadurch gekennzeichnet, daß** sie als niederviskoses Epoxid gemäß Komponente b) 3,4-Epoxycyclohexylmethyl-3,4-epoxycyclohexancarboxylat, 3,4-Epoxy-6-methylcyclohexyl-3,4-epoxy-6-methyl-cyclohexancarboxylat, Dicyclopentadiendioxid, Bis(3,4-epoxycyclohexylnethyl)adipinat, 1,3,5,7-Tetrakis(2,1-ethandiyl-3,4-epoxycyclohexyl)-1,3,5,7-tetramethylcyclotetrasiloxan, 1,3,5,7,9-Pentakis(2,1-ethandiyl-3,4-epoxycyclohexyl)-1,3,5,7,9-pentamethylcyclopentasiloxan, 1,1,3,3-Tetramethyl-1,3-bis(ethandiyl-3,4-epoxycyclohexyl)disiloxan und/oder niedermolekulare Siloxane, die mit cycloaliphatischen Epoxiden funktionalisiert sind, enthält.

4. Polymerisierbare Masse nach mindestens einem der Patentansprüche 1 bis 3, **dadurch gekennzeichnet, daß** sie als Füllstoffe gemäß Komponente c) Quarz, gemahlene Gläser, Kieselgele oder Kieselsäuren oder deren Granulate enthält.

5. Polymerisierbare Masse nach mindestens einem der Patentansprüche 1 bis 4, **dadurch gekennzeichnet, daß** sie als Verzögerer, Beschleuniger und/oder Initiatoren Lewis-Säuren oder Broensted-Säuren oder Verbindungen, aus denen durch Bestrahlen mit UV- oder sichtbarem Licht, durch Wärme und/oder Druck solche Säuren entstehen, enthält.

6. Polymerisierbare Masse nach mindestens einem der Patentansprüche 1 bis 5, **dadurch gekennzeichnet, daß** sie als Hilfsstoffe Diole, Verdünnungsmittel, Stabilisatoren, Inhibitoren und/oder Pigmente enthält.

7. Polymerisierbare Masse nach mindestens einem der Patentansprüche 1 bis 6, bestehend aus
A einer basispaste, enthaltend die Epoxide der Komponenten (a) und (b), einen Teil oder dem gesamten Füllstoff der Komponente (c), gegebenenfalls Verzögerer oder Beschleuniger gemäß Komponente (d) und gegebenenfalls Hilfsstoffe der Komponente (e), sowie räumlich getrennt hiervon
B einer Katalysatorpaste, enthaltend einen Initiator gemäß Komponente (d), gegebenenfalls einen Teil des Füllstoffs der Komponente (c) und gegebenenfalls Hilfsstoffe gemäß Komponente (e).

8. Epoxide, nämlich
i) 2,2-Bis[4,1-phenylenoxy-3,1-propandiyl-3,4-epoxycyclohexylcarbonsäureester]propyliden 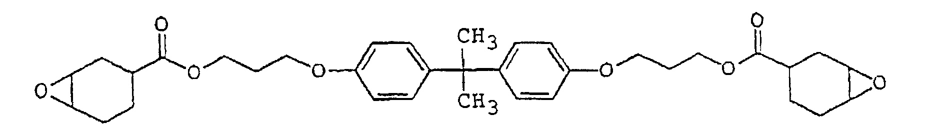
ii) 2,2-Bis[4,1-phenylenoxy-3,1-propandiyl-oxymethandiyl-3,4-epoxycyclohexyl]propyliden 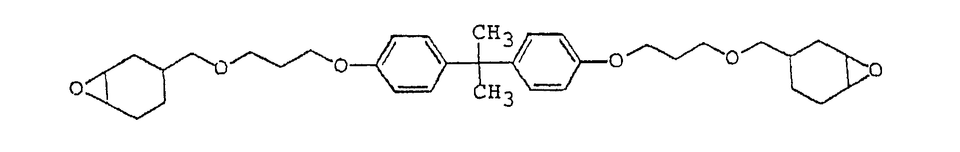
iii) 2,2-Bis[3,4-epoxycyclohexylmethandiyl(4,1-phenylenoxy-3,1-propylcarbonsäureester)]propyliden 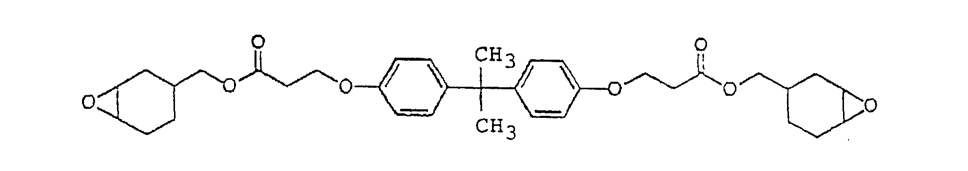
iv) 2,2-Bis[4,1-phenylenoxy-3,1-propandiyl-1,1,3,3-tetramethyldisiloxanyl-1,2-ethandiyl-3,4-epoxycyclohexyl]propyliden 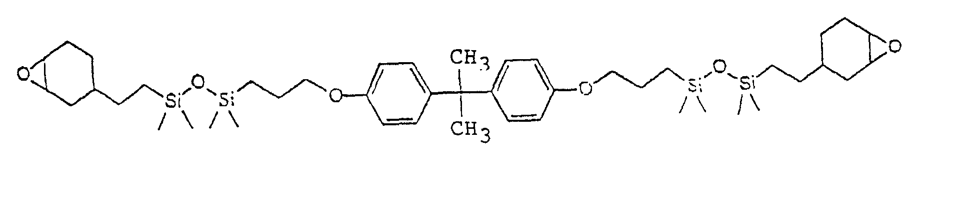
v) 2,2-Bis{4,1-phenylenoxy-3,1-propandiyl-3-oxatricyclo[3.2.1.0^{2,4}]octyl-6-carboxy}propyliden 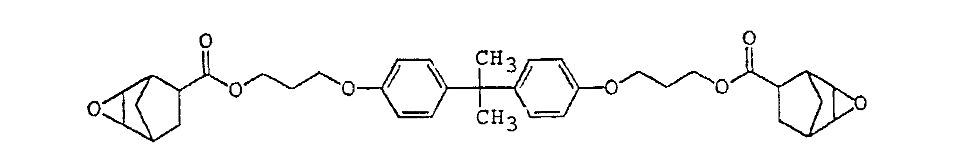
vi) 2,2-Bis{4,1-phenylenoxy-3,1-propandiyl-3,8-dioxatricyclo[3.2.1.0^{2,4}]octyl-6-carboxy}-propyliden 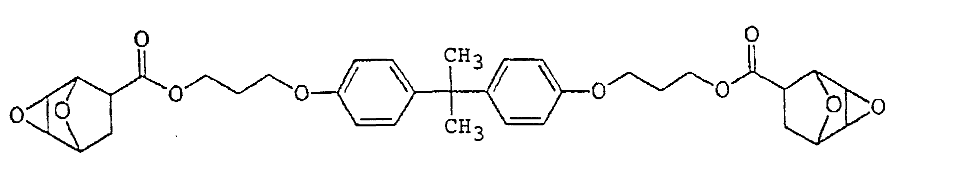
vii) 2,2-Bis{4,1-phenylenoxy-3,1-propandiyl-[3,5,7-tris(ethandiyl-3,4-epoxycyclohexyl)-1,3,5,7-tetramethylcyclotetrasiloxanyl]}propyliden 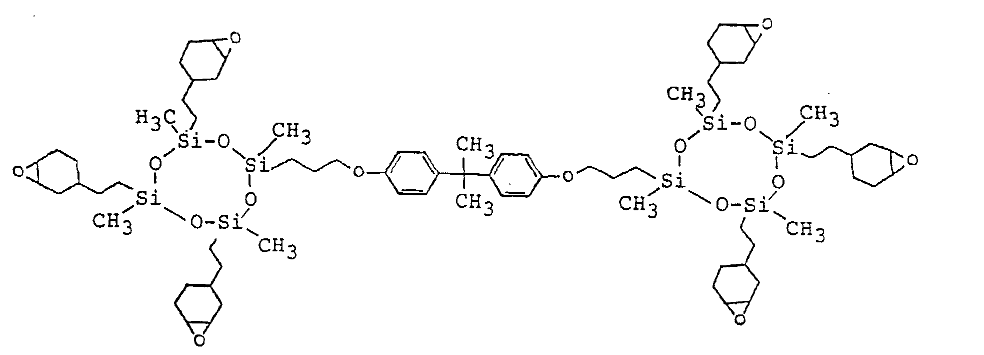
viii) Bis[methandiyl-oxy-3,1-propandiyl-3,4-epoxycyclohexylcarbonsäureester]tricyclo[5.2.1.0^{2,6}]-decan 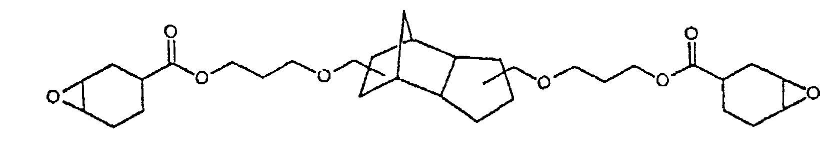
ix) Bis[methandiyl-oxy-3,1-propandiyl-oxy-methandiyl-3,4-epoxycyclohexyl]tricyclo[5.2.1.0^{2,6}]-decan 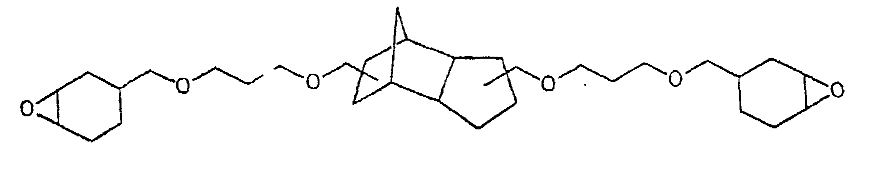
x) Bis[3,4-epoxycyclohexylmethandiyl-propancarbonsäure-1-oxy-methandiyl]tricyclo[5.2.1.0^{2,6}]-decan 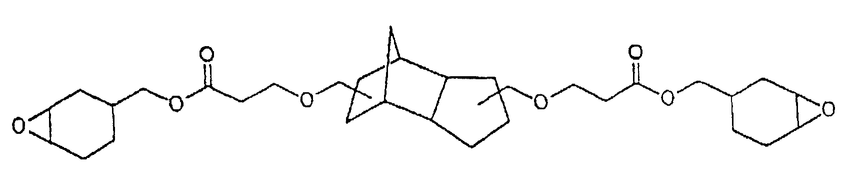
xi) Bis(methandiyl-oxy-3,1-propandiyl-1,1,3,3-tetramethyldisiloxandiyl-1,2-ethandiyl-3,4-epoxycyclohexyl]tricyclo[5.2.1.0^{2,6}]decan 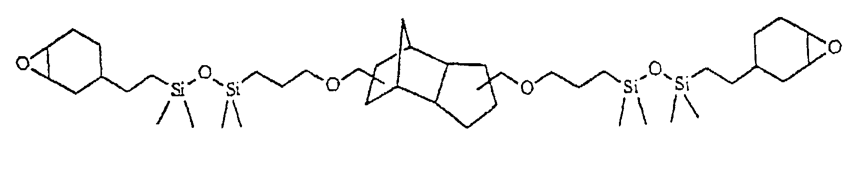
xii) Bis{methandiyl-oxy-3,1-propandiyl-3-oxatricyclo[3.2.1.0^{2,6}]octyl-6-carboxyl}tricyclo-[5.2.1.0^{2,6}]decan 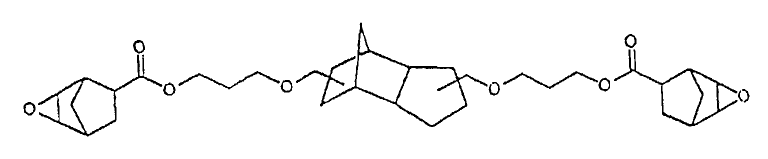
xiii) Bis{methandiyl-oxy-3,1-propandiyl-3,8-dioxatricyclo[3.2.1.0^{2,6}]octyl-6-carboxyl}tricyclo-[5.2.1.0^{2,6}]decan 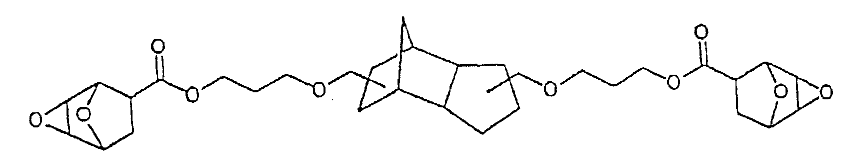
xiv) Bis(methandiyl-oxy-(3-propandiyl-3,5,7-tris(2,1-ethandiyl-3,4-epoxycyclohexyl)-1,3,5,7-tetramethylcyclotetrasiloxanyl)-tricyclo[5.2.1.0^{2,6}]-decan 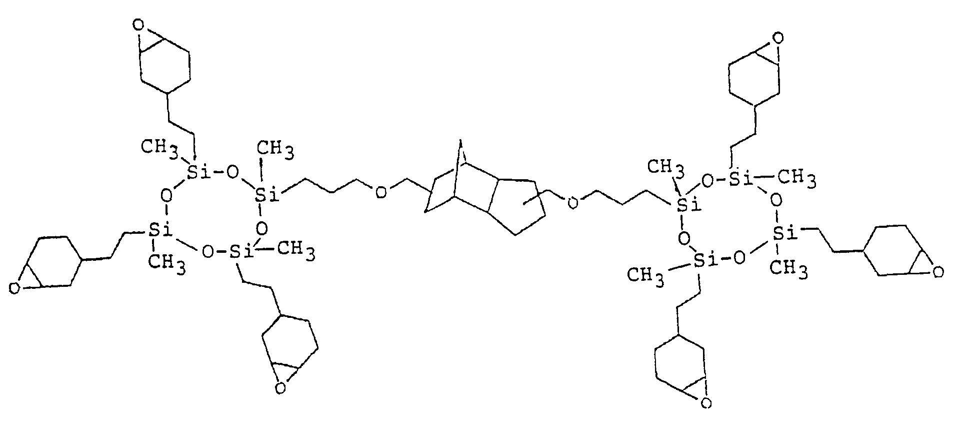
xv) 1,1,1-Tris[methandiyl-oxy-methandiyl-3,4-epoxycyclohexyl]propan 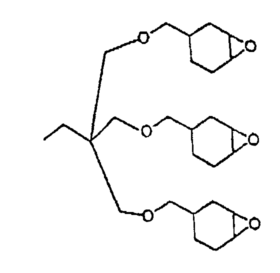
xvi) 1,1,1-Tris[methandiyl-oxy-1,3-propandiyl-1,1,3,3-tetramethyldisiloxandiyl-1,2-ethandiyl-3,4-epoxycyclohexyl]propan 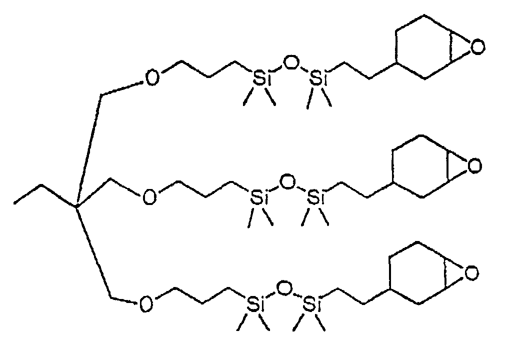
xvii) 1,1,1-Tris{methandiyl-3-oxatricyclo-[3.2.1.0^{2,4}]octyl-6-carboxy}propan 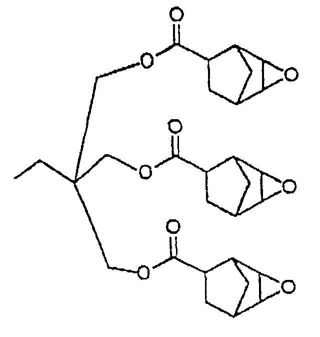
xviii) 1,1,1-Tris{methandiyl-3,8-dioxatricyclo-[3.2.1.0^{2,4}]octyl-6-carboxy}propan 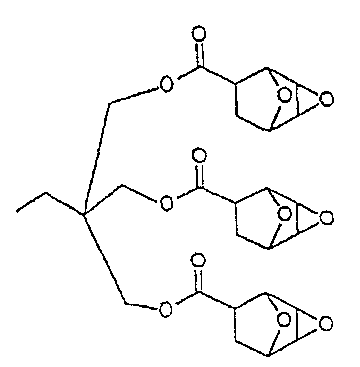
xix) 1,1,1-Tris[methandiyl-oxy-3,1-propandiyl-3,5,7-tris(2,1-ethandiyl-3,4-epoxycyclohexyl)-1,3,5,7-tetramethylcyclotetrasiloxanyl]propan 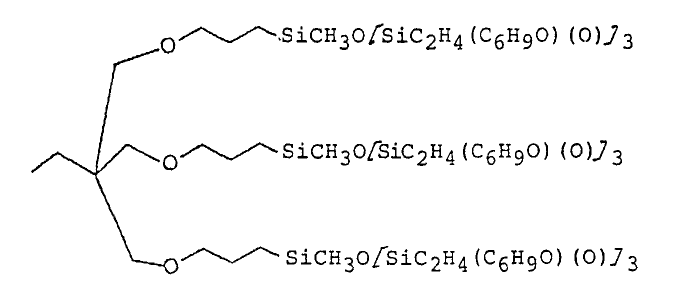
xx) 1,1,1-Tris[methandiyl-oxy-bis(ethandiyloxy)-3,4-epoxycyclohexylcarbonsäureester]propan 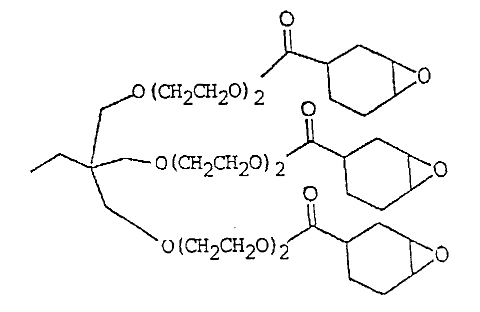
xxi) 1,1,1-Tris[methandiyl-oxy-bis(ethandiyloxy)-methandiyl-3,4-epoxycyclohexyl]propan 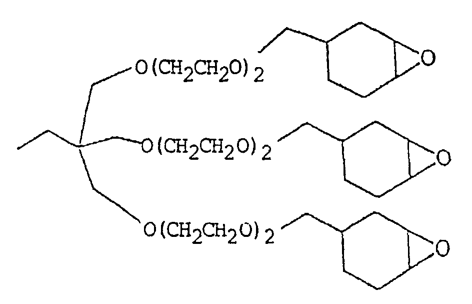
xxii) 1,1,1-Tris[methandiyl-oxy-bis(ethandiyloxy)-propandiyl-1,1,3,3-tetramethyldisiloxanyl-1,2-ethandiyl-3,4-epoxycyclohexyl]propan 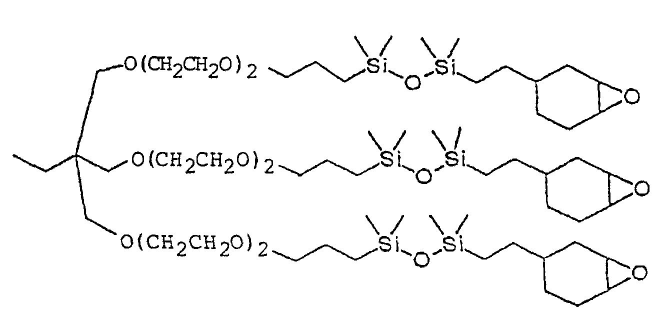
xxiii) 1,1,1-Tris{methandiyl-oxy-bis(ethandiyloxy)-3-oxatricyclo[3.2.1.0^{2,4}]octyl-6-carboxy}propan 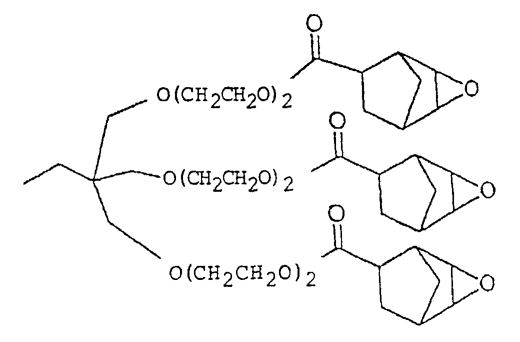
xxiv) 1,1,1-Tris{methandiyl-oxy-bis(ethandiyloxy)-3,8-dioxatricyclo[3.2.1.0^{2,4}]octyl-6-carboxy}propan 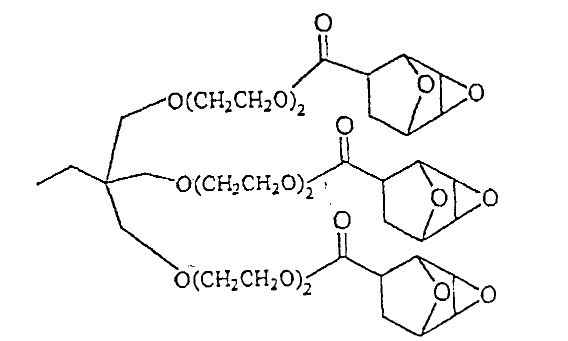
xxv) 1,1,1-Tris[methandiyl-oxy-bis(ethandiyloxy)-propandiyl-3,5,7-tris(2,1-ethandiyl-3,4-epoxycyclohexyl)-1,3,5,7-tetramethylcyclotetrasiloxa nyl]propan 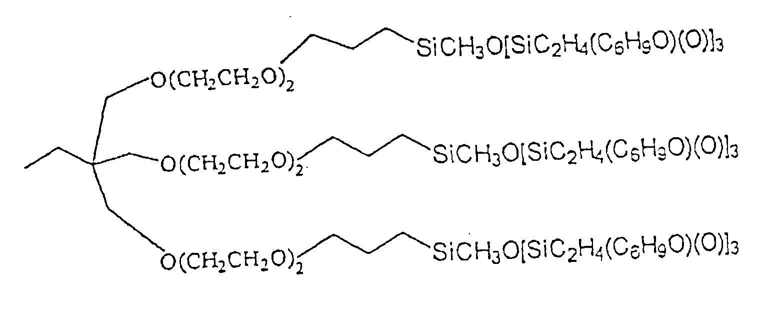
xxvi) α,ω-Bis[3,4-epoxycyclohexylethandiyl-1,1,3,3-tetramethyldisiloxanyl-3,1-propandiyl]polytetrahydrofuran 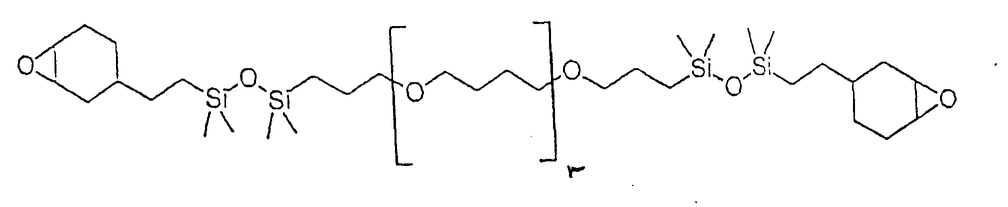
xxvii) α,ω-Bis{3-oxatricyclo[3.2.1.0^{2,4}]octyl-6-carboxy}polytetrahydrofuran 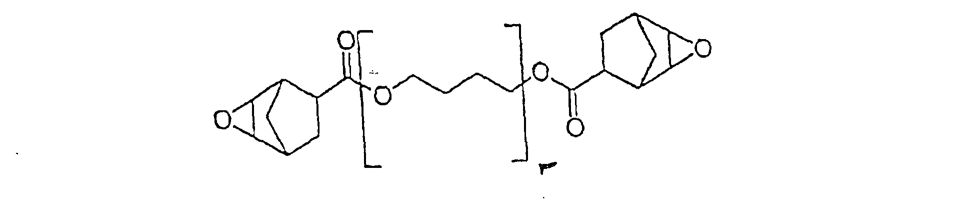
xxviii) α,ω-Bis{3,8-dioxatricyclo[3.2.1.0^{2,4}]octyl-6-carboxy}polytetrahydrofuran 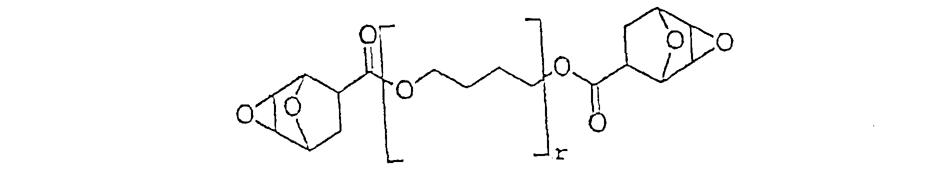
xxix) α,ω-Bis-(3-propandiyl-3,5,7-tris(2,1-ethandiyl-3,4-epoxycyclohexyl)-1,3,5,7-tetramethylcyclotetrasiloxanyl)polytetrahydrofuran

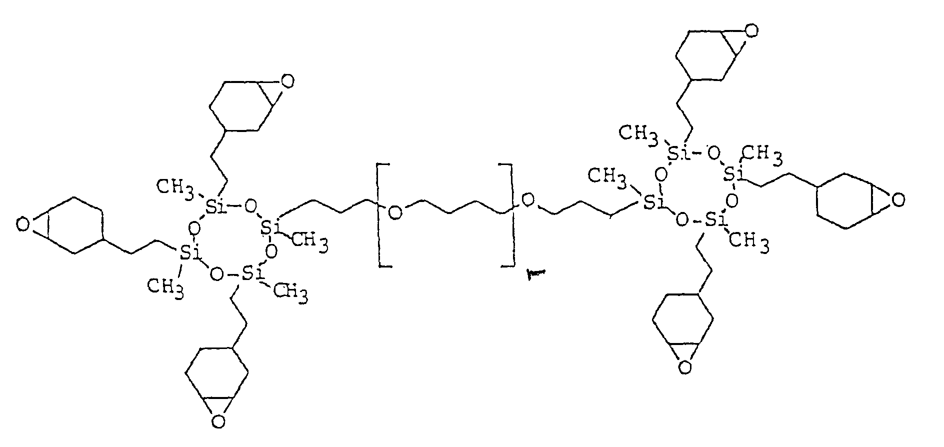

9. Verwendung der polymerisierbaren Massen nach mindestens einem der Patentansprüche 1 bis 7 oder der cycloaliphatischen Epoxide nach Patentanspruch 8 als Beschichtungsmittel.

10. Verwendung der polymerisierbaren Massen nach mindestens einem der Patentansprüche 1 bis 7 oder der cycloaliphatischen Epoxide nach Patentanspruch 8 zum Verkleben von Substraten.

11. Verwendung der polymerisierbaren Massen nach mindestens einem der Patentansprüche 1 bis 7 oder der cycloaliphatischen Epoxide nach Patentanspruch 8 als Dentalmassen.

## Claims

1. Polymerizable composition comprising
(a) 3 to 80 wt.% of an epoxide or a mixture of epoxides of the general formula: 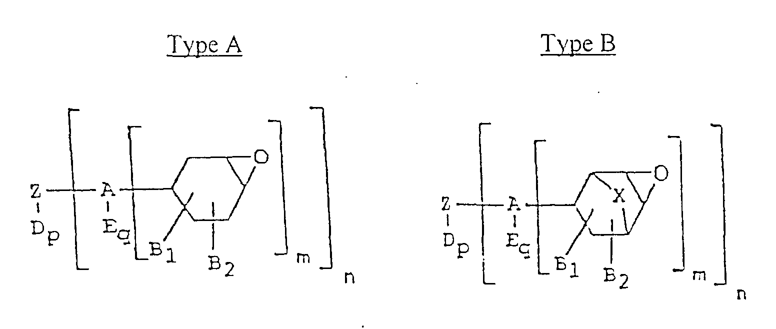 in which, for type A:
if n=2
Z denotes a cycloaliphatic or aromatic radical having 1 to 22 C atoms or a combination of these radicals, wherein one or more C atoms can be replaced by O, C=O, -O(C=O)-, SiR₂ and/or NR, or an aliphatic radical having 0 to 22 C atoms, wherein one or more C atoms can be replaced by O, C=O, -O(C=O)-, NR or SiR₂, wherein in the case of an aliphatic radical at least one C atom must be replaced by SiR₂, and wherein R is an aliphatic radical having 1 to 7 C atoms, wherein one or more C atoms can be replaced by O, C=O and/or -O(C=O)-,
if n>2
Z denotes an aliphatic, cycloaliphatic or aromatic radical having 0 to 22 C atoms or a combination of these radicals, wherein one or more C atoms can be replaced by O, C=O, -O(C=O)-, SiR₂ and/or NR and wherein R is an aliphatic radical having 1 to 7 C atoms, wherein one or more C atoms can be replaced by O, C=O and/or -O(C=O)-,
and in which, for type B:
Z denotes an aliphatic, cycloaliphatic or aromatic radical having 0 to 22 C atoms or a combination of these radicals, wherein one or more C atoms can be replaced by O, C=O, -O(C=O)-, SiR₂ and/or NR and wherein R is an aliphatic radical having 1 to 7 C atoms, wherein one or more C atoms can be replaced by O, C=O and/or -O(C=O)-,
and in which, for type A and type B:
A denotes an aliphatic, cycloaliphatic or aromatic radical having 1 to 18 C atoms or a combination of these radicals, wherein one or more C atoms can be replaced by O, C=O, -O(C=O)-, SiR₂ and/or NR, wherein R is an aliphatic radical having 1 to 7 C atoms, in which one or more C atoms can be replaced by O, C=O and/or -O(C=O)-,
B₁, B₂, D and E independently of one another denote an H atom or an aliphatic radical having 1 to 9 C atoms, wherein one or more C atoms can be replaced by O, C=O, -O(C=O)-, SiR₂ and/or NR, wherein R is an aliphatic radical having 1 to 7 C atoms, in which one or more C atoms can be replaced by O, C=O and/or-O(C=O)-,
n denotes 2-7,
m denotes 1-10,
p denotes 1-5,
q denotes 1-5 and
x denotes CH₂, S or O,
(b) 0 to 80 wt.% of an epoxide or a mixture of epoxides which differ from (a),
(c) 3 to 85 wt.% of fillers,
(d) 0.01 to 25 wt.% of initiators, retardants and/or accelerators,
(e) 0 to 25 wt.% of auxiliaries,
the percentage data in each case being based on the total weight of the composition.

2. Polymerizable composition as claimed in patent claim 1, **characterized in that** it comprises, as component (a), one or more of the following epoxides:
i) 2,2-bis[4,1-phenylenoxy-3,1-propanediyl-3,4-epoxycyclohyexylcarboxylic acid ester]propylidene 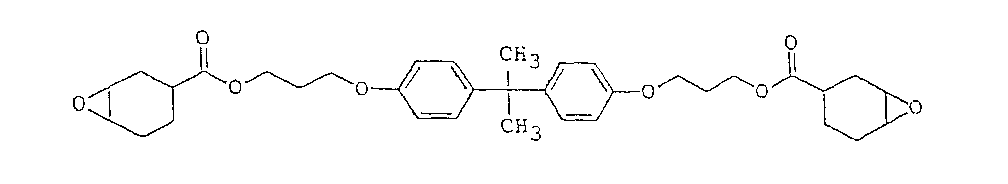
ii) 2,2-bis[4,1-phenylenoxy-3,1-propanediyl-oxy-methanediyl-3,4-epoxycyclohexyl]propylidene 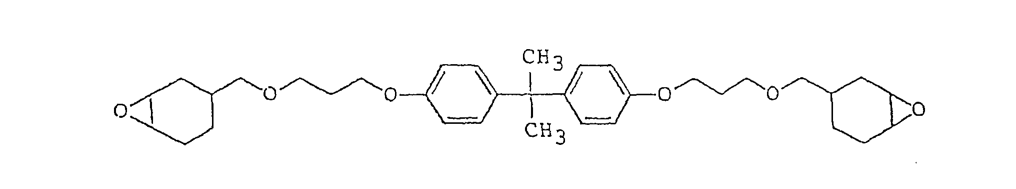
iii) 2,2-bis[3,4-epoxycyclohexylmethanediyl(4,1-phenylenoxy-3,1-propylcarboxylic acid ester)]propylidene 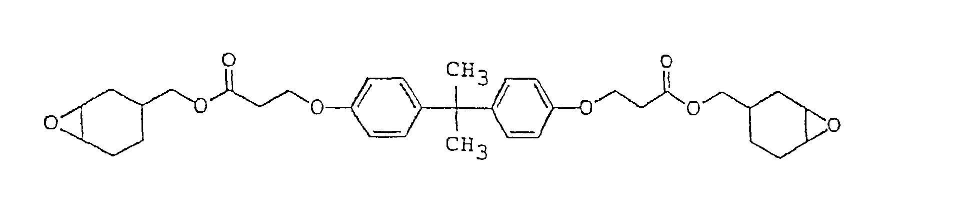
iv) 2,2-bis[4,1-phenylenoxy-3,1-propanediyl-1,1,3,3-tetramethyldisiloxanyl-1,2-ethanediyl-3,4-epoxycyclohexyl]propylidene 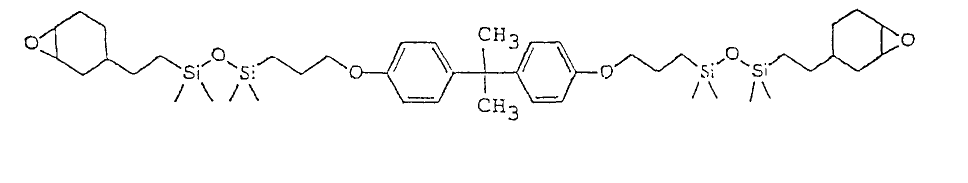
v) 2,2-bis {4,1-phenylenoxy-3,1-propanediyl-3-oxatricyclo[3.2.1.0^{2,4]}octyl-6-carboxy}propylidene 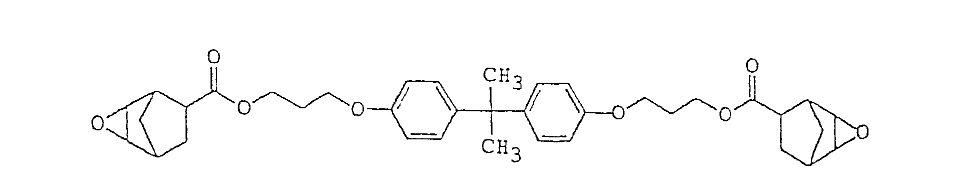
vi) 2,2-bis{4,1-phenylenoxy-3,1-propanediyl-3,8-dioxatricyclo[3,2,1.0^{2,4}]octyl-6-carboxy}propylidene 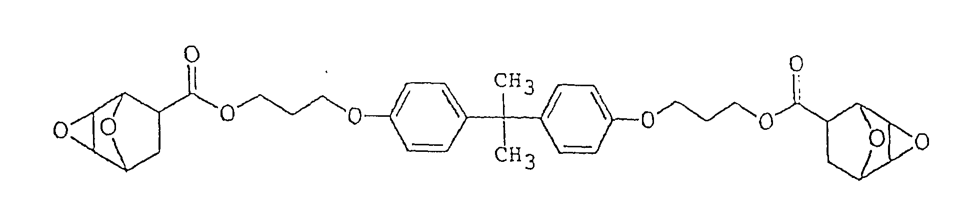
vii) 2,2-bis{4,1-phenylenoxy-3,1-propanediyl-[3,5,7-tris(ethanediyl-3,4-epoxycyclohexyl)-1,3,5,7-tetramethylcyclotetrasiloxanyl]}propylidene 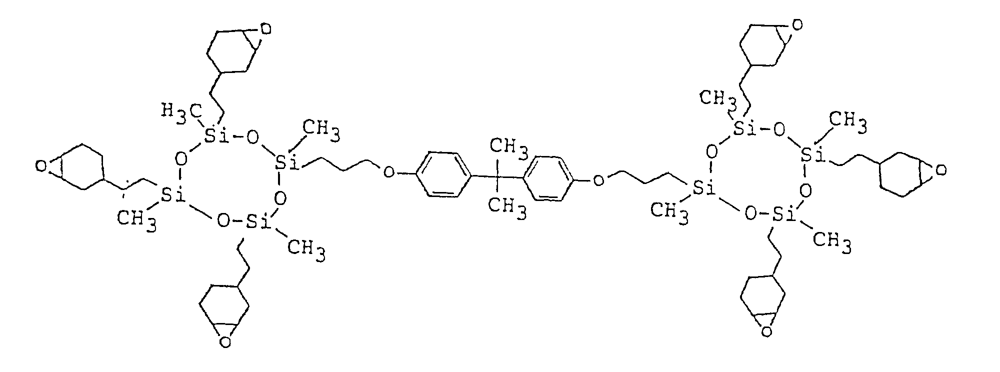
viii) bis[methanediyl-oxy-3,1-propanediyl-3,4-epoxycyclohyexylcarboxylic acid ester]tricyclo[5.2.1.0^{2,6}]decane 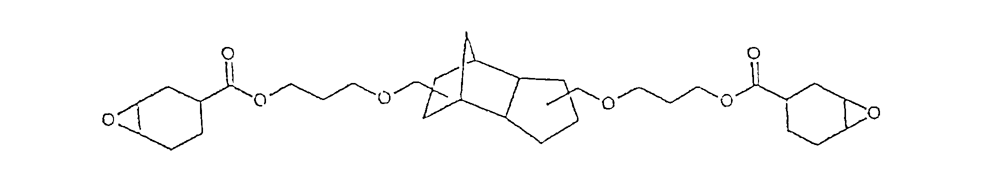
ix) bis[methanediyl-oxy-3,1-propanediyl-oxy-methanediyl-3,4-epoxycyclohexyl]tricyclo[5.2.1.0^{2,6}]decane 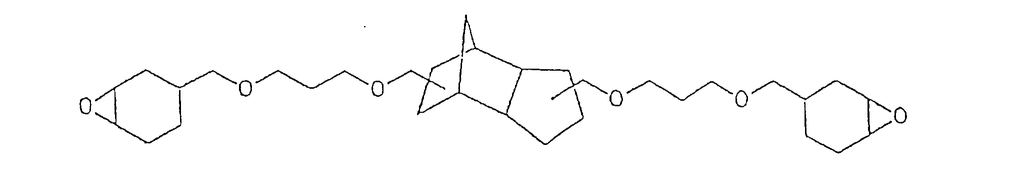
x) bis[3,4-epoxycyclohexylmethanediyl-propanecarboxylic acid-1-oxymethanediyl]tricyclo[5.2.1.0^{2,6}]decane 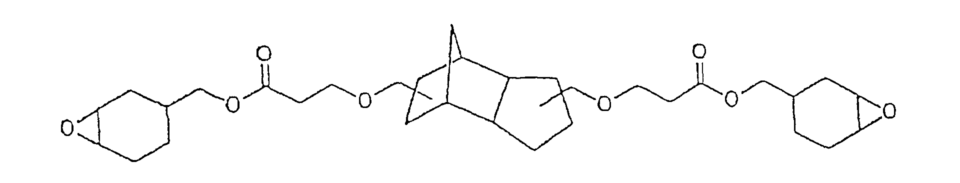
xi) bis(methanediyl-oxy-3,1-propanediyl-1,1,3,3-tetramethyldisiloxanediyl-1,2-ethanediyl-3,4-epoxycyclohexyl)tricyclo[5.2.1.0^{2,6}]decane 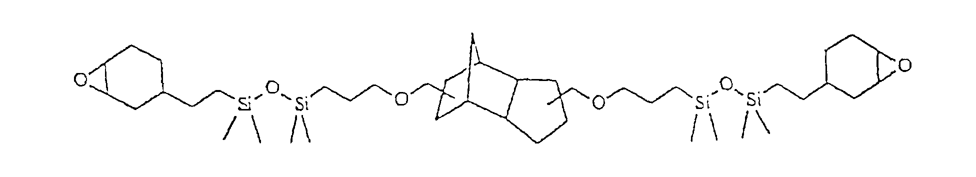
xii) bis{methanediyl-oxy-3,1-propanediyl-3-oxatricyclo[3.2.1.0^{2,6}]octyl-6-carboxyl}tricyclo[5.2.1.0^{2,6}]decane 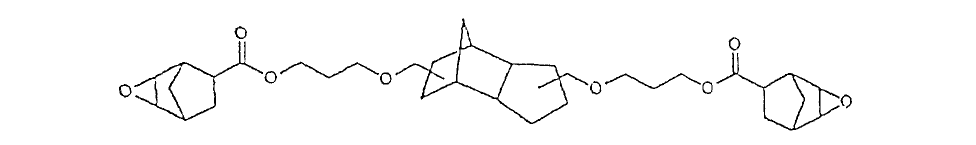
xiii) bis{methanediyl-oxy-3,1-propanediyl-3,8-dioxatricyclo[3.2.1.0^{2,6}]octyl-6-carboxyl}tricyclo[5.2.1.0^{2,6}]decane 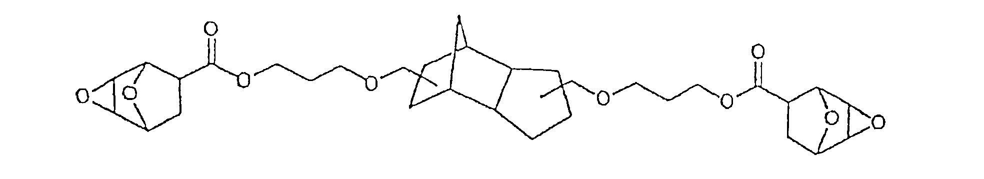
xiv) bis{methanediyl-oxy-(3-propanediyl-3,5,7-tris(2,1-ethanediyl-3,4-epoxycyclohexyl)-1,3,5,7-tetramethylcyclotetrasiloxanyl)-tricyclo[5.2.1.0^{2,6}]decane 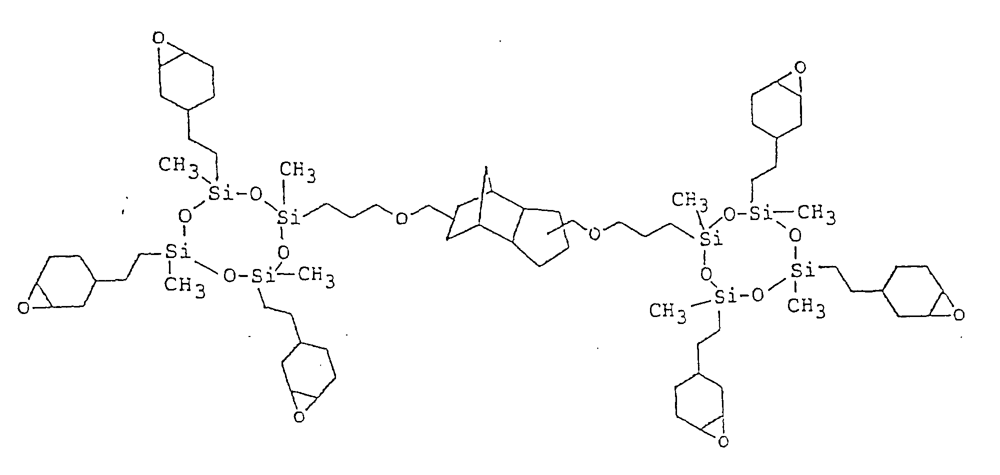
xv) 1,1,1-tris[methanediyl-oxy-methanediyl-3,4-epoxycyclohexyl]propane 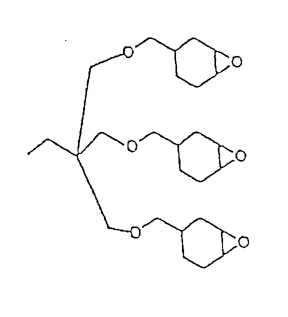
xvi) 1,1,1-tris[methanediyl-oxy-1,3-propanediyl-1,1,3,3-tetramethyldisiloxanediyl-1,2-ethanediyl-3,4-epoxycyclohexyl]propane 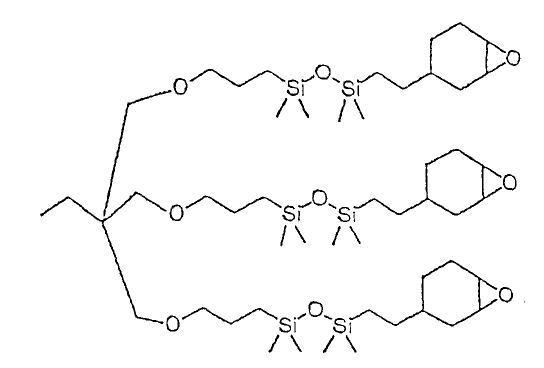
xvii) 1,1,1-tris{methanediyl-3-oxatricyclo[3.2.1.0^{2,4}]octyl-6-carboxy}propane 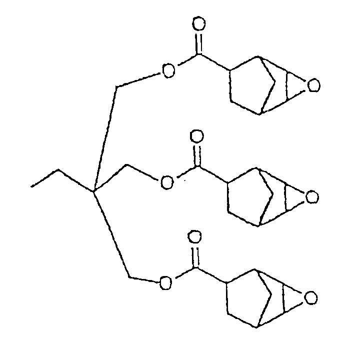
xviii) 1,1,1-tris{methanediyl-3,8-dioxatricyclo[3.2.1.0^{2,4}]octyl-6-carboxy}propane 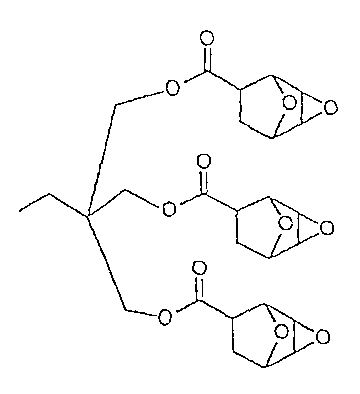
xix) 1,1,1-tris{methanediyl-oxy-3,1-propanediyl-3,5,7-tris(2,1-ethanediyl-3,4-epoxycyclohexyl)-1,3,5,7-tetramethylcyclotetrasiloxanyl] propane 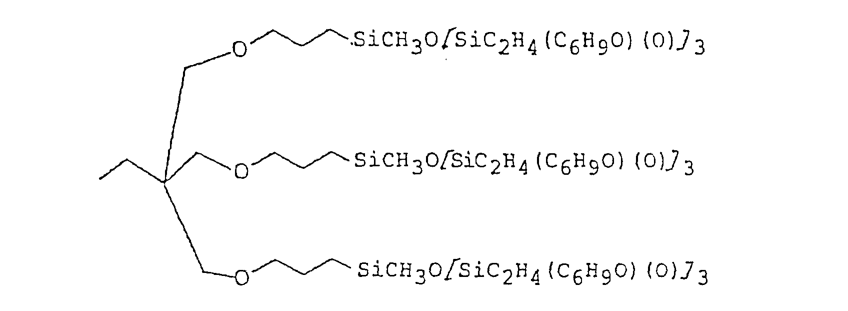
xx) 1,1,1-tris[methanediyl-oxy-bis(ethanediyloxy)-3,4-epoxycyclohexylcarboxylic acid ester]propane 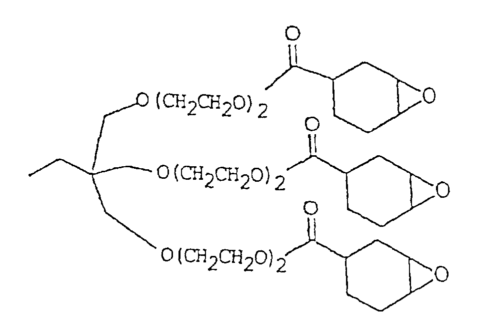
xxi) 1,1,1-tris[methanediyl-oxy-bis(ethanediyloxy)-methanediyl-3,4-epoxycyclohexyl]propane 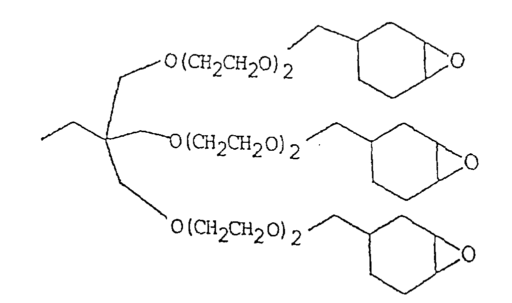
xxii) 1,1,1-tris[methanediyl-oxy-bis(ethanediyloxy)-propanediyl-1,1,3,3-tetramethyldisiloxanyl-1,2-ethanediyl-3,4-epoxycyclohexyl]propane 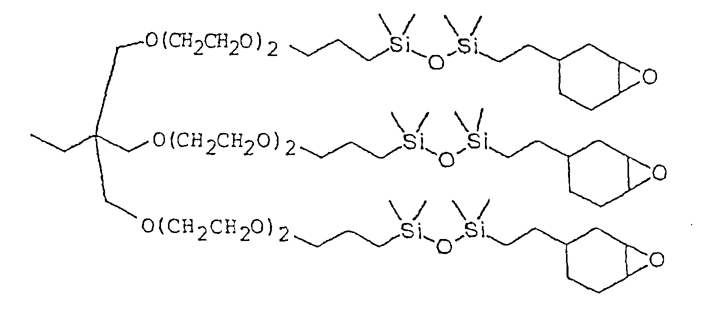
xxiii) 1,1,1-tris{methanediyl-oxy-bis(ethanediyloxy)-3-oxatricyclo[3.2.1.0^{2,4}]octyl-6-carboxy}propane 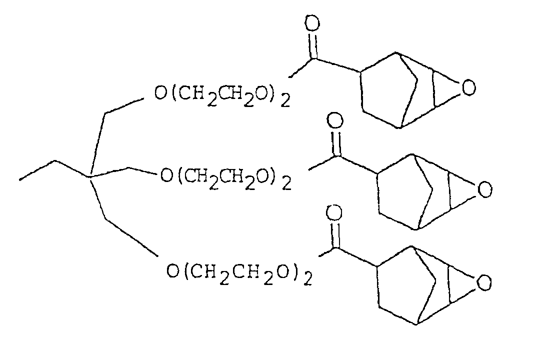
xxiv) 1,1,1-tris{methanediyl-oxy-bis(ethanediyloxy)-3,8-dioxatricyclo[3.2.1.0^{2,4}]octyl-6-carboxy}propane 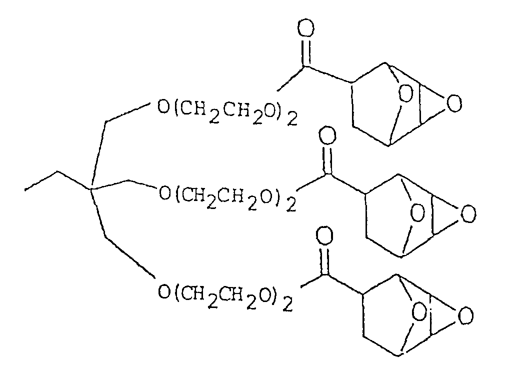
xxv) 1,1,1-tris[methanediyl-oxy-bis(ethanediyloxy)-propanediyl-3,5,7-tris(2,1-ethanediyl-3,4-epoxycyclohexyl)-1,3,5,7-tetramethylcyclotetrasiloxanyl]propane 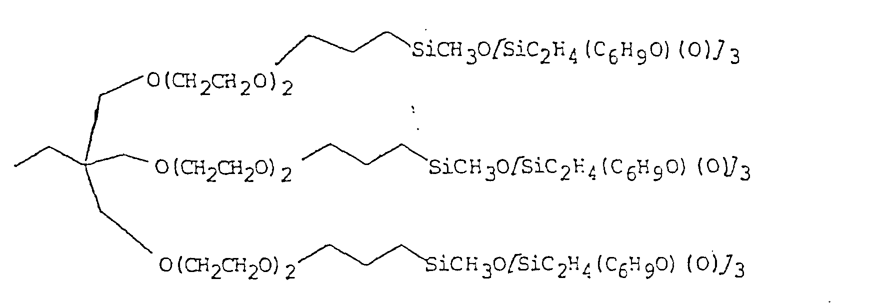
xxvi) α,ω-bis[3,4-epoxycyclohexylethanediyl-1,1,3,3-tetramethyldisiloxanyl-3,1-propanediyl]polytetrahydrofuran 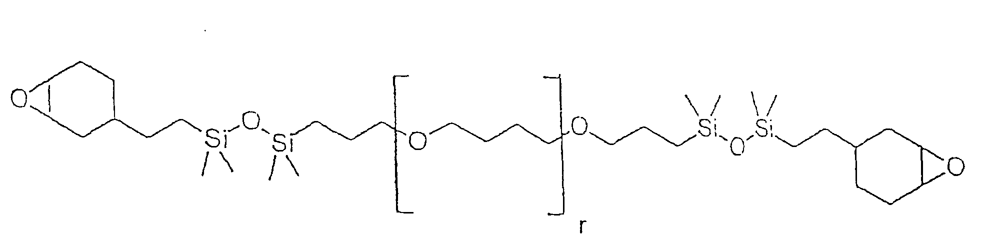
xxvii) α,ω-bis{3-oxatricyclo[3.2.1.0^{2.4}]octyl-6-carboxy}polytetrahydrofuran 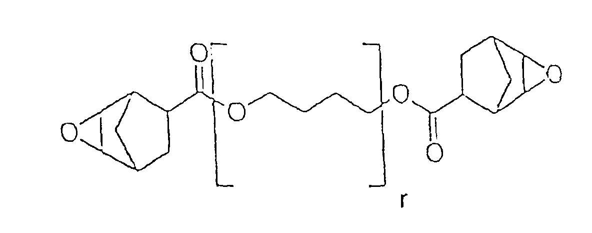
xxviii) α,ω-bis{3,8-dioxatricyclo[3.2.1.0^{2,4}]octyl-6-carboxy}polyletrahydrofuran 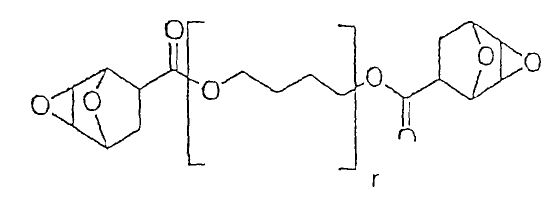
xxix) α,ω-bis(3-propanediyl-3,5,7-tris(2,1-ethanediyl-3,4-epoxycyclohexyl)-1,3,5,7-tetramethylcyclotetrasiloxanyl)polytetrahydrofuran

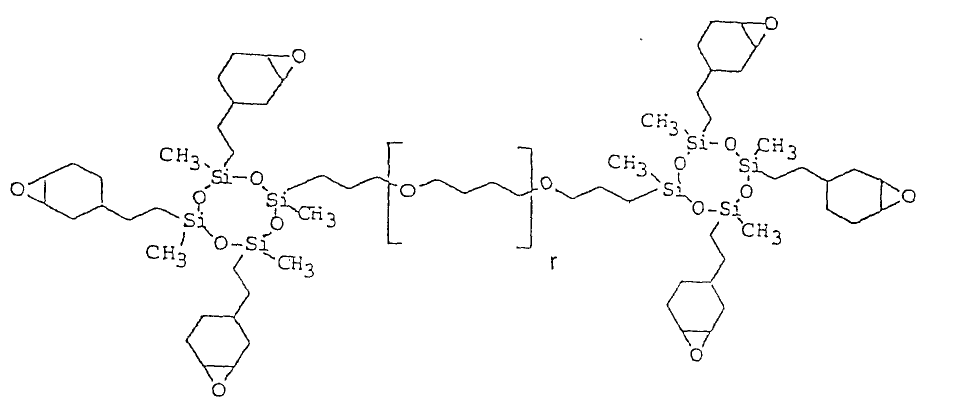

3. Polymerizable composition as claimed in patent claim 1 or 2, **characterized in that** it comprises, as the low-viscosity epoxide according to component b) 3,4-epoxycyclohexylmethyl 3,4-epoxycyclohexanecarboxylate, 3,4-epoxy-6-methylcyclohexyl 3,4-epoxy-6-methylcyclohexanecarboxylate, dicyclopentadiene dioxide, bis(3,4-epoxycyclohexylmethyl) adipinate, 1,3,5,7-tetrakis(2,1-ethanediyl-3,4-epoxycyclohexyl)-1,3,5,7-tetramethylcyclotetrasiloxane, 1,3,5,7,9-pentakis(2,1-ethanediyl-3,4-epoxycyclohexyl)-1,3,5,7,9-pentamethylcyclopentasiloxane, 1,1,3,3-tetramethyl-1,3-bis(ethanediyl-3,4-epoxycyclohexyl)disiloxane and/or low molecular weight siloxanes which are functionalized with cycloaliphatic epoxides.

4. Polymerizable composition according to at least one of patent claims 1 to 3, **characterized in that** it comprises, as fillers according to component c), quartz, ground glasses, silica gels or silicic acids or granules thereof.

5. Polymerizable composition according to at least one of patent claims 1 to 4, **characterized in that** it comprises, as retardants, accelerators and/or initiators, Lewis acids or Broensted acids or compounds from which such acids are formed by irradiation with UV light or visible light or by heat and/or pressure.

6. Polymerizable composition according to at least one of patent claims 1 to 5, **characterized in that** it comprises, as auxiliaries, diols, diluents, stabilizers, inhibitors and/or pigments.

7. Polymerizable composition according to at least one of patent claims 1 to 6, comprising
A a base paste comprising the epoxides of components (a) and (b), a portion or all of the filler of component (c), if appropriate retardants or accelerators according to component (d) and if appropriate auxiliaries of component (e), and, spatially separated therefrom.
B a catalyst paste comprising an initiator according to component (d), if appropriate a portion of the filler of component (c) and if appropriate auxiliaries according to component (e).

8. Epoxides, namely
i) 2,2-bis[4,1-phenylenoxy-3,1-propanediyl-3,4-epoxycyclohyexylcarboxylic acid ester]propylidene 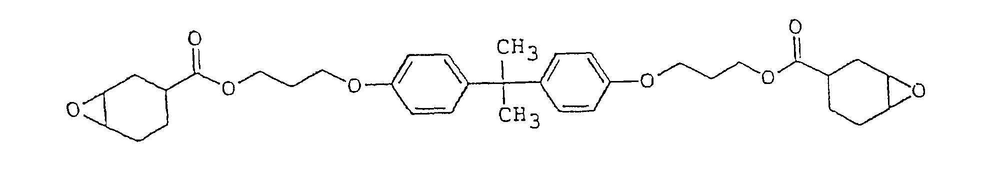
ii) 2,2-bis[4,1-phenylenoxy-3,1-propanediyl-oxy-methanediyl-3,4-epoxycyclohexyl]propylidene 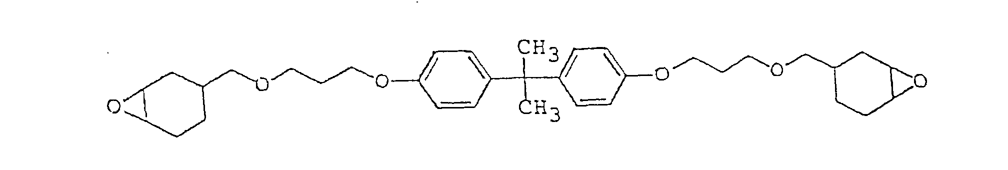
iii) 2,2-bis[3,4-epoxycyclohexylmethanediyl(4,1-phenylenoxy-3,1-propylcarboxylic acid ester)]propylidene 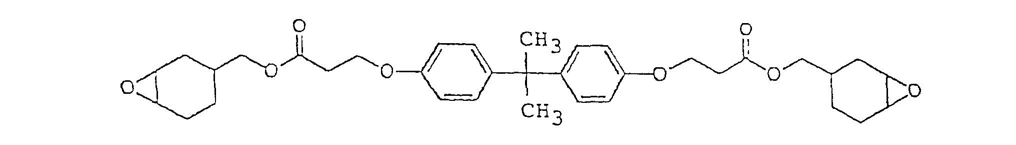
iv) 2,2-bis[4,1-phenylenoxy-3,1-propanediyl-1,1,3,3-tetramethyldisiloxanyl-1,2-ethanediyl-3,4-epoxycyclohexyl]propylidene 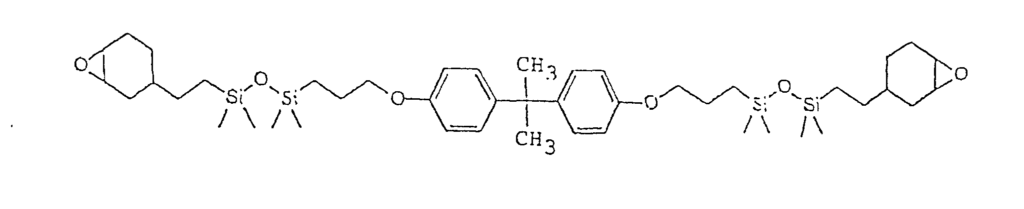
v) 2,2-bis{4,1-phenylenoxy-3,1-propanediyl-3-oxatricyclo[3.2.1.0^{2,4]}octyl-6-carboxy} propylidene 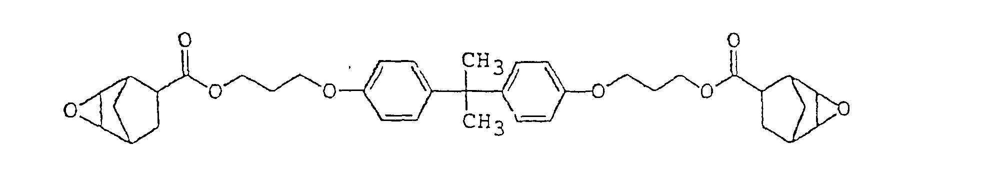
vi) 2,2-bis{4,1-phenylenoxy-3,1-propanediyl-3,8-dioxatricyclo[3.2.1.0^{2,4}]octyl-6-carboxy}propylidene 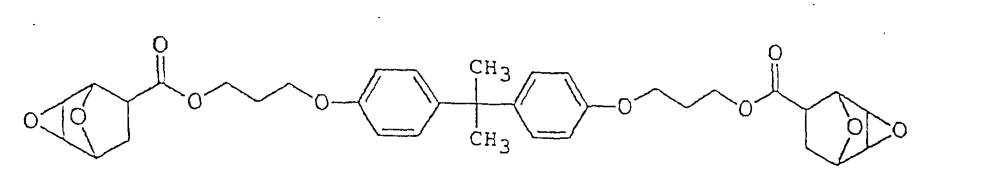
vii) 2,2-bis{4,1-phenylenoxy-3,1-propanediyl-[3,5,7-tris(ethanediyl-3,4-epoxycyclohexyl)-1,3,5,7-tetramethylcyclotetrasiloxanyl]}propylidene 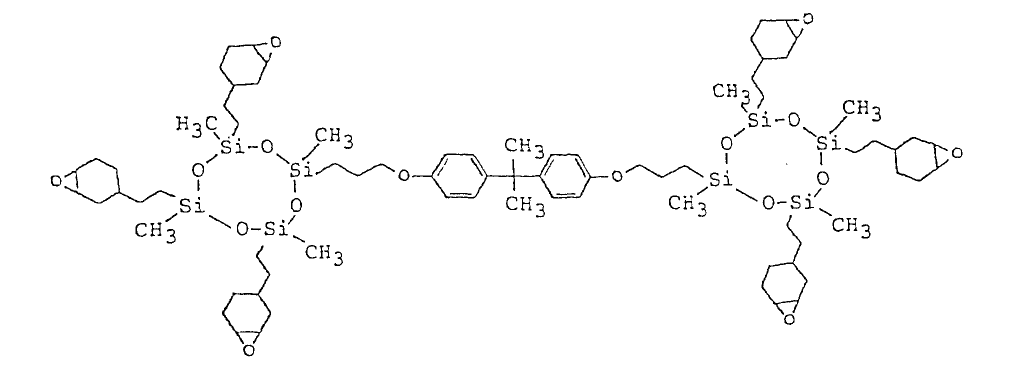
viii) bis[methanediyl-oxy-3,1-propanediyl-3,4-epoxycyclohyexylcarboxylic acid ester]tricyclo[5.2.1.0^{2,6}]decane 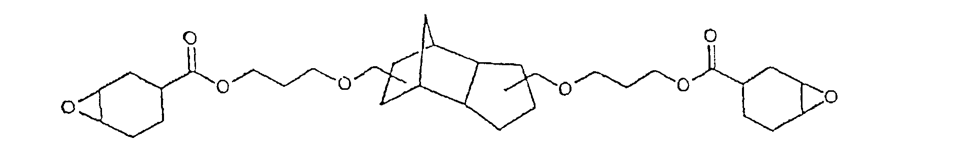
ix) bis[methanediyl-oxy-3,1-propanediyl-oxy-methanediyl-3,4-epoxycyclohexyl]tricyclo[5,2,1.0^{2,6}]decane 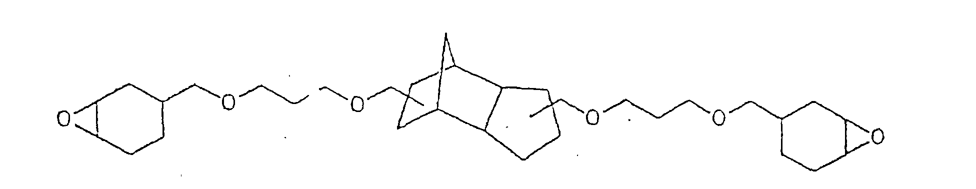
x) bis[3,4-epoxycyclohexylmethanediyl-propanecarboxylic acid-1-oxymethanediyl]tricyclo[5.2.1 0^{2,6}]decane 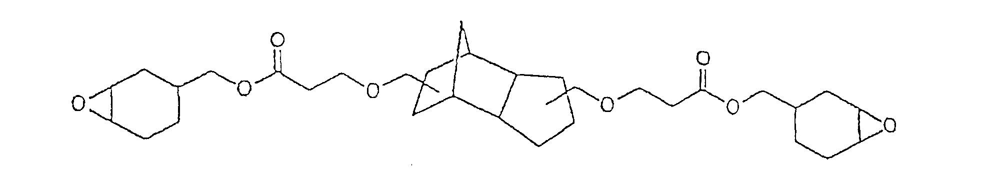
xi) bis(methanediyl-oxy-3,1-propanediyl-1,1,3,3-tetramethyldisiloxanediyl-1,2-ethanediyl-3,4-epoxycyclohexyl)tricyclo[5.2.1.0^{2,6}]decane 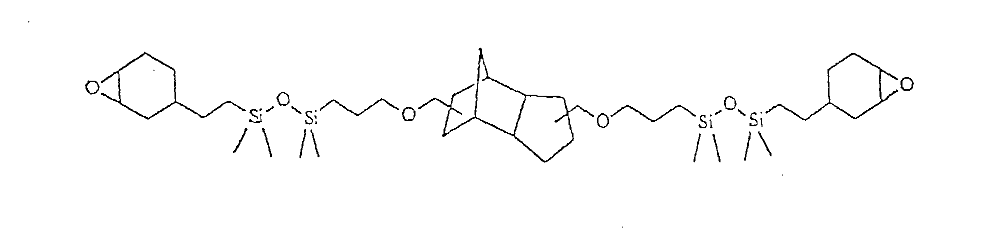
xii) bis{methanediyl-oxy-3,1-propanediyl-3-oxatricyclo[3.2.1.0^{2,6}]octyl-6-carboxyl}tricyclo[5.2.1.0^{2,6}]decane 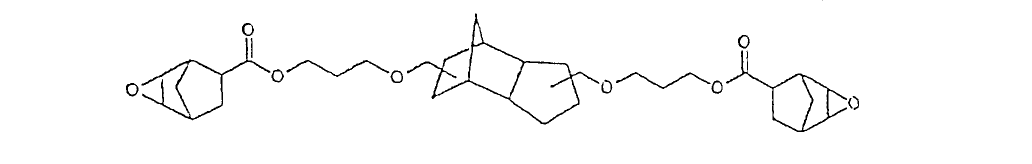
xiii) bis{methanediyl-oxy-3,1-propanediyl-3,8-dioxatricyclo[3,2,1.0^{2,6}]octyl-6-carboxyl}tricyclo[5.2.1.0^{2,6}]decane 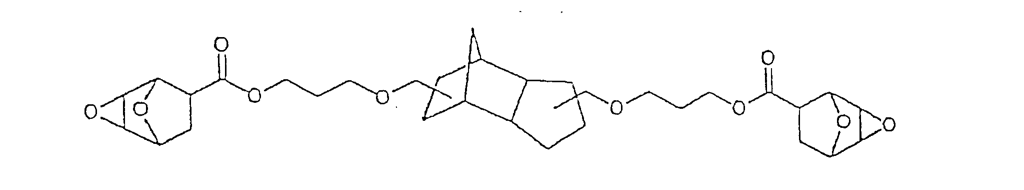
xiv) bis {methanediyl-oxy-(3-propanediyl-3,5,7-tris(2,1-ethanediyl-3,4-epoxycyclohexyl)-1,3,5,7-tetramethylcyclotetrasiloxanyl)-tricyclo[5.2.1.0^{2,6}]decane 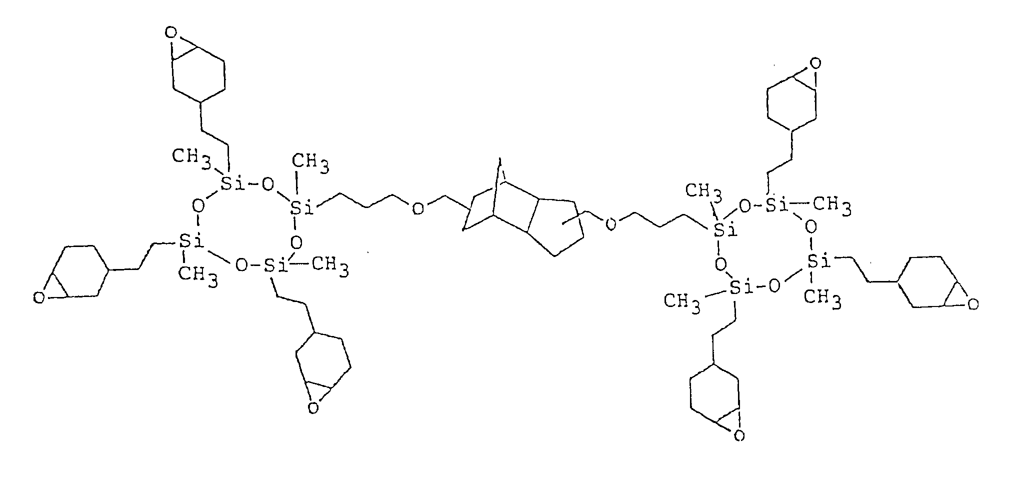
xv) 1,1,1-tris[methanediyl-oxy-methanediyl-3,4-epoxycyclohexyl]propane 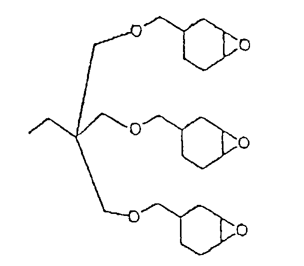
xvi) 1,1,1-tris[methanediyl-oxy-1,3-propanediyl-1,1,3,3-tetramethyldisiloxanediyl-1,2-ethanediyl-3,4-epoxycyclohexyl]propane 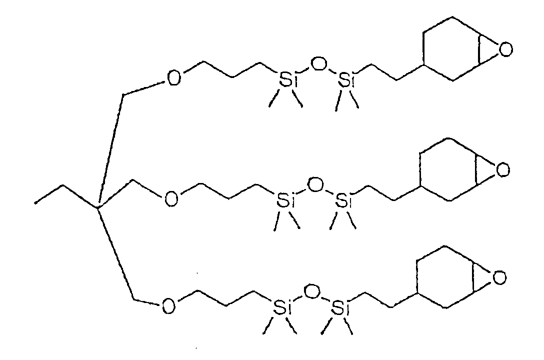
xvii) 1,1,1-tris{methanediyl-3-oxatricyclo[3.2.1.0^{2,4}]octyl-6-carboxy}propane 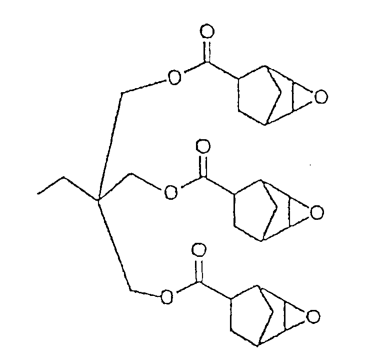
xviii) 1,1,1-tris{methanediyl-3,8-dioxatricyclo[3.2.1.0^{2,4}]octyl-6-carboxy}propane 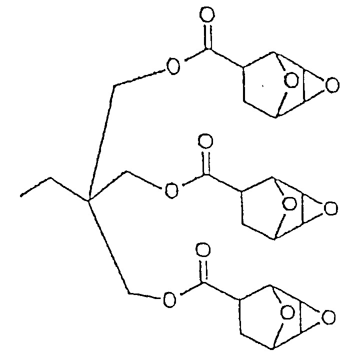
xix) 1,1,1-tris{methanediyl-oxy-3,1-propanediyl-3,5,7-tris(2,1-ethanediyl-3,4-epoxycyclohexyl)-1,3,5,7-tetramethylcyclotetrasiloxanyl]propane 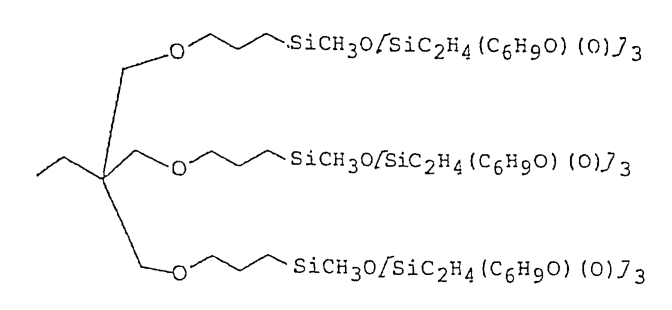
xx) 1,1,1-tris[methanediyl-oxy-bis(ethanediyloxy)-3,4-epoxycyclohexylcarboxylic acid ester]propane 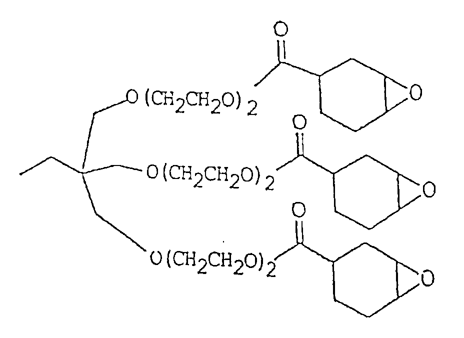
xxi) 1,1,1-tris[methanediyl-oxy-bis(ethanediyloxy)-methanediyl-3,4-epoxycyclohexyl]propane 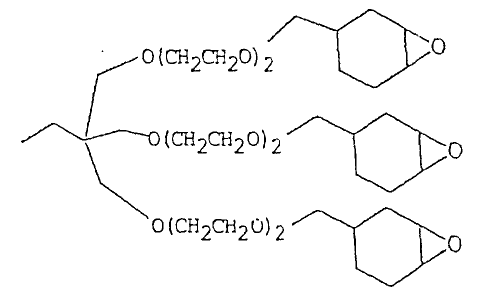
xxii) 1,1,1-tris[methanediyl-oxy-bis(ethanediyloxy)-propanediyl-1,1,3,3-tetramethyldisiloxanyl-1,2-ethanediyl-3,4-epoxycyclohexyl]propane 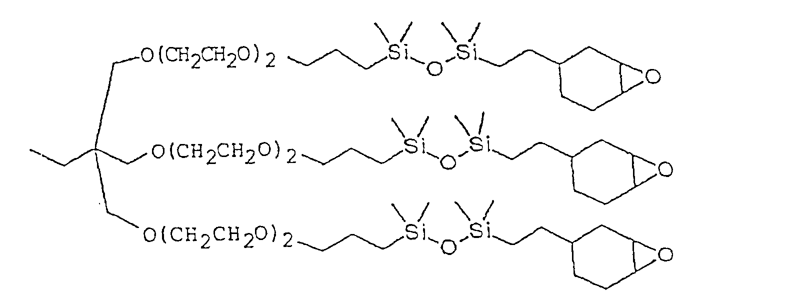
xxiii) 1,1,1-tris{methanediyl-oxy-bis(ethanediyloxy)-3-oxatricyclo[3.2.1.0^{2,4}]octyl-6-carboxy}propane 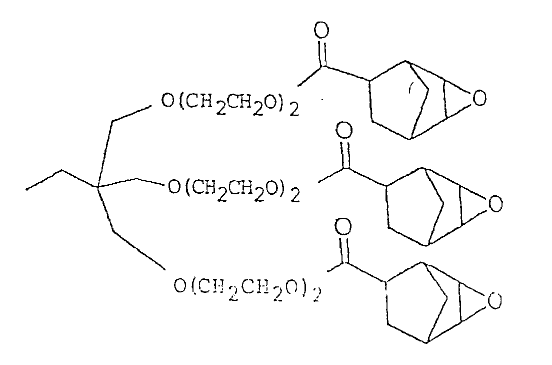
xxiv) 1,1,1-tris{methanediyl-oxy-bis(ethanediyloxy)-3,8-dioxatricyclo[3.2.1.0^{2,4}]octyl-6-carboxy} propane 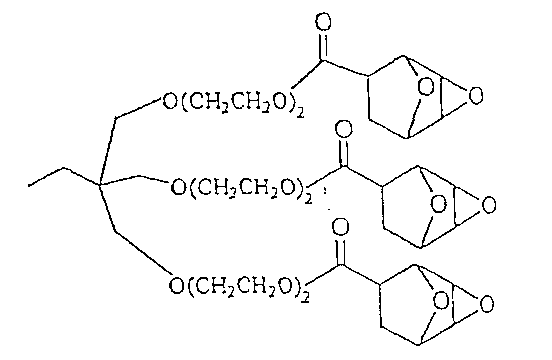
xxv) 1,1,1-tris[methanediyl-oxy-bis(ethanediyloxy)-propanediyl-3,5,7-tris(2,1-ethanediyl-3,4-epoxycyclohexyl)-1,3,5,7-tetramethylcyclotetrasiloxanyl]propane 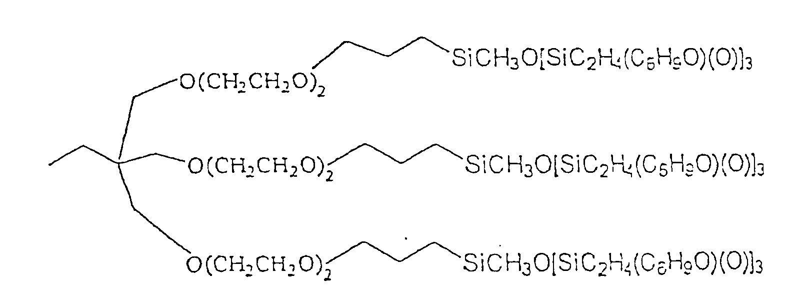
xxvi) α,ω-bis[3,4-epoxycyclohexylethanediyl-1,1,3,3-tetramethyldisiloxanyl-3,1-propanediyl]polytetrahydrofuran 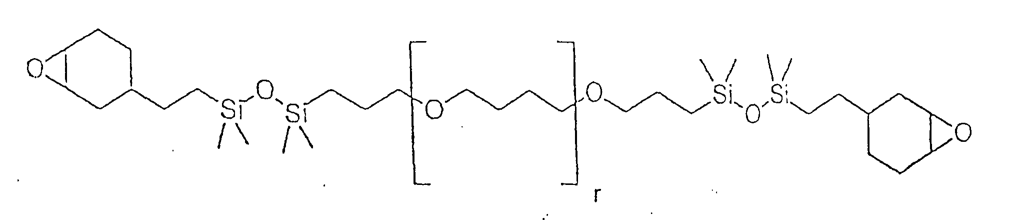
xxvii) α,ω-bis{3-oxatricyclo[3.2.1.0^{2,4}]octyl-6-carboxy}polytetrahydrofuran 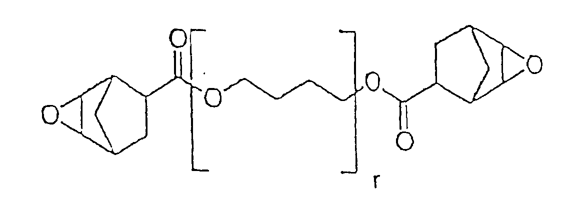
xxviii) α,ω-bis{3,8-dioxatricyclo[3.2.1.0^{2,4}]octyl-6-carboxy}polytetrahydrofuran 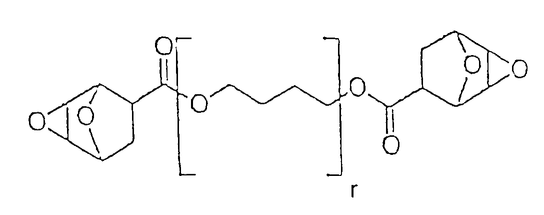
xxix) α,ω-bis(3-propanediyl-3,5,7-tris(2,1-ethanediyl-3,4-epoxycyclohexyl)-1,3,5,7-tetramethylcyclotetrasiloxanyl)polytetrahydrofuran

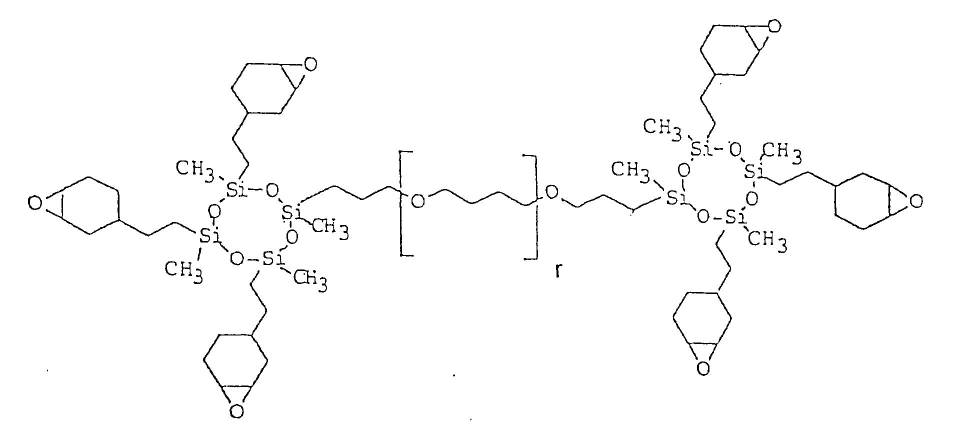

9. Use of the polymerizable compositions according to at least one of patent claims 1 to 7 or of the cycloaliphatic epoxides according to patent claim 8 as coating compositions.

10. Use of the polymerizable compositions according to at least one of patent claims 1 to 7 or of the cycloaliphatic epoxides according to patent claim 8 for gluing substrates.

11. Use of the polymerizable compositions according to at least one of patent claims 1 to 7 or of the cycloaliphatic epoxides according to patent claim 8 as dental compositions.

## Revendications

1. Composition polymérisable, contenant
(a) de 3 à 80% en poids d'un époxyde ou d'un mélange d'époxydes de la formule générale : 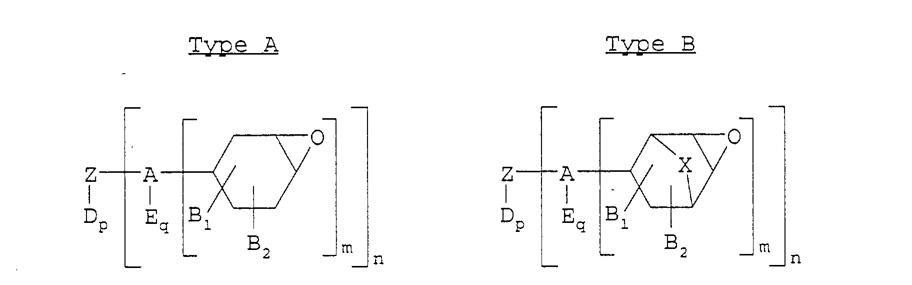 dans laquelle, pour le type A :
lorsque n = 2 :
Z est un résidu cycloaliphatique ou aromatique ayant de 1 à 22 atomes de carbone ou une combinaison de ces résidus, un ou plusieurs atomes de carbone pouvant être remplacés par O, C=O, -O(C=O)-, SiR₂ et/ou NR, ou bien un résidu aliphatique ayant de 0 à 22 atomes de carbone, un ou plusieurs atomes de carbone pouvant être remplacés par O, C=O, -O(C=O)-, NR ou SiR₂, au moins un atome de carbone devant être remplacé par SiR₂ dans le cas d'un résidu aliphatique, R étant un résidu aliphatique ayant de 1 à 7 atomes de carbone dans lequel un ou plusieurs atomes de carbone peuvent être remplacés par O, C=O et/ou -O(C=O)-,
lorsque n > 2 :
Z est un résidu aliphatique, cycloaliphatique ou aromatique ayant de 0 à 22 atomes de carbone ou une combinaison de ces résidus, un ou plusieurs atomes de carbone pouvant être remplacés par O, C=O, -O(C=O)-, SiR₂ et/ou NR, où R est un résidu aliphatique ayant de 1 à 7 atomes de carbone dans lequel un ou plusieurs atomes de carbone peuvent être remplacés par O, C=O et/ou -O(C=O)-,
et dans laquelle, pour le type B :
Z est un résidu aliphatique, cycloaliphatique ou aromatique ayant de 0 à 22 atomes de carbone ou une combinaison de ces résidus, un ou plusieurs atomes de carbone pouvant être remplacés par O, C=O, -O(C=O)-, SiR₂ et/ou NR, où R est un résidu aliphatique ayant de 1 à 7 atomes de carbone dans lequel un ou plusieurs atomes de carbone peuvent être remplacés par O, C=O et/ou -O(C=O)-,
et dans laquelle, pour le type A et le type B :
A est un résidu aliphatique, cycloaliphatique ou aromatique ayant de 1 à 18 atomes de carbone ou une combinaison de ces résidus, un ou plusieurs atomes de carbone pouvant être remplacés par O, C=O, -O(C=O)-, SiR₂ et/ou NR, où R est un résidu aliphatique ayant de 1 à 7 atomes de carbone dans lequel un ou plusieurs atomes de carbone peuvent être remplacés par O, C=O et/ou -O(C=O)-,
B₁, B₂, D, E sont indépendamment les uns des autres un atome d'hydrogène ou un résidu aliphatique ayant de 1 à 9 atomes de carbone, un ou plusieurs atomes de carbone pouvant être remplacés par O, C=O, -O(C=O)-, SiR₂ et/ou NR, où R est un résidu aliphatique ayant de 1 à 7 atomes de carbone dans lequel un ou plusieurs atomes de carbone peuvent être remplacés par O, C=O et/ou -O(C=O)-,
n vaut de 2 à 7,
m vaut de 1 à 10,
p vaut de 1 à 5,
q vaut 1 à 5 et
X est CH₂, S ou O,
(b) de 0 à 80% en poids d'un époxyde ou d'un mélange d'époxydes différents de (a),
(c) de 3 à 85% en poids de matières de charge
(d) de 0,01 à 25% en poids d'amorces, de retardateurs et/ou d'accélérateurs,
(e) de 0 à 25% en poids d'adjuvants, les pourcentages indiqués se rapportant toujours au poids total de la composition.

2. Composition polymérisable selon la revendication 1, **caractérisée en ce qu'**elle contient en tant que composant (a) un ou plusieurs des époxydes suivants :
i) 2,2-bis[4,1-phénylénoxy-3,1-propanediyl-3,4-époxycyclohexylcarboxylate]propylidène 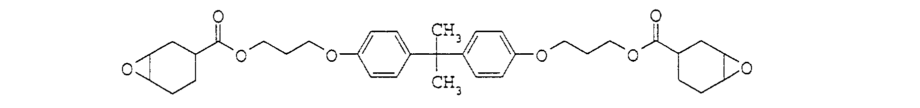
ii) 2,2-bis[4,1-phénylénoxy-3,1-propanediyloxyméthanediyl-3,4-époxycyclohexyl]propylidène 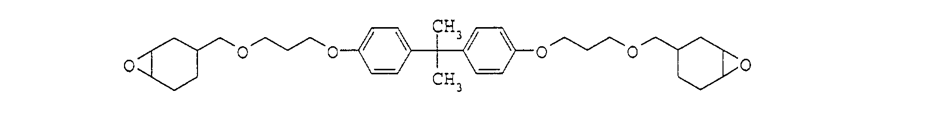
iii) 2,2-bis[3,4-époxycyclohexylméthanediyl(4,1-phénylénoxy-3,1-propylcarboxylate)]propylidène 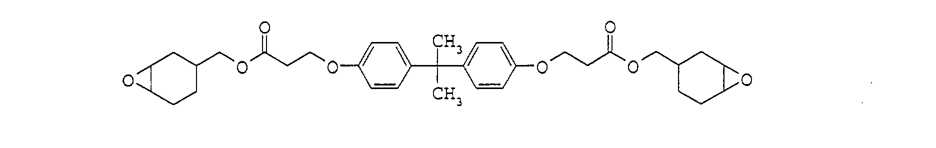
iv) 2,2-bis[4,1-phénylénoxy-3,1-propanediyl-1,1,3,3-tétraméthyldisiloxanyl-1,2-éthanediyl- 3,4-époxycyclohexyl]propylidène 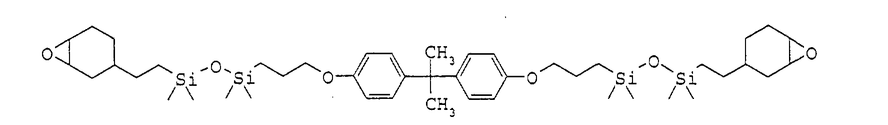
v) 2,2-bis{4,1-phénylénoxy-3,1-propanediyl-3-oxa- tricyclo[3.2.1.0^{2,4}]octyl-6-carboxy}propylidène 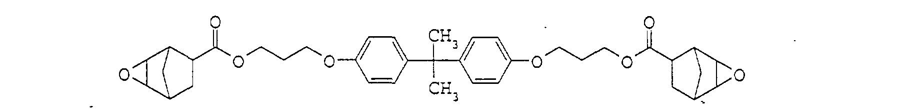
vi) 2,2-bis{4,1-phénylénoxy-3,1-propanediyl-3,8-dioxatricyclo[3.2.1.0^{2,4}]octyl-6-carboxy}-propylidène 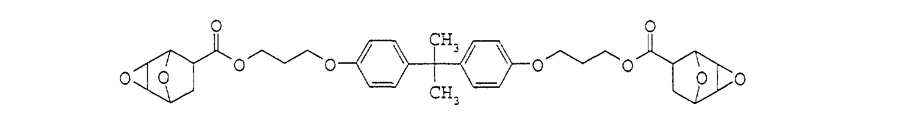
vii) 2,2-bis{4,1-phénylénoxy-3,1-propanediyl-[3,5,7- tris(éthanediyl-3,4-époxycyclohexyl)-1,3,5,7-tétraméthylcyclotétrasiloxanyl]}propylidène 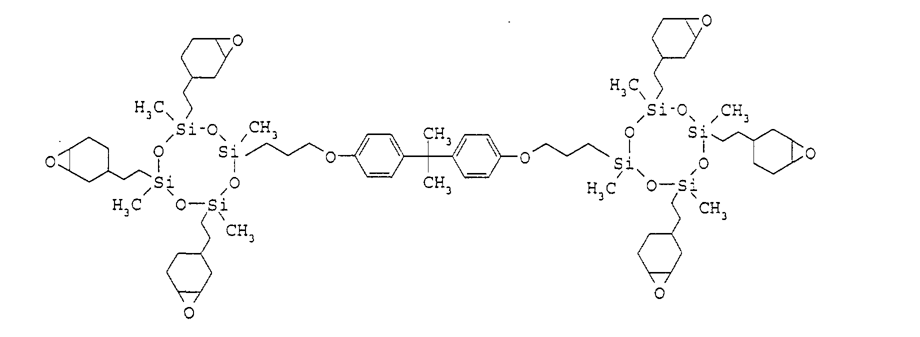
viii) bis[méthanediyloxy-3,1-propanediyl-3,4-époxy- cyclohexylcarboxylate]tricyclo[5.2.1.0^{2,6}]décane 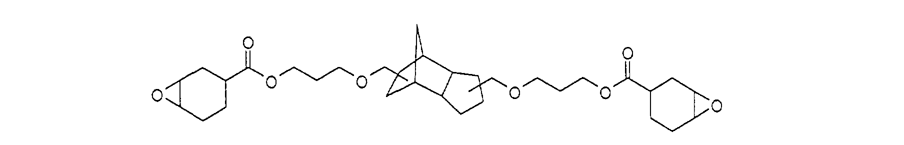
ix) bis[méthanediyloxy-3,1-propanediyloxyméthanediyl-3,4-époxycyclohexyl]tricyclo[5.2.1.0^{2,6}]- décane 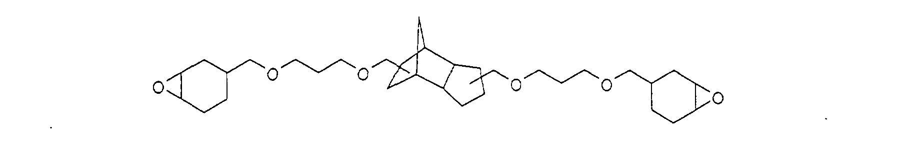
x) bis[3,4-époxycyclohexylméthanediylpropanecarboxylate-1-oxyméthanediyl]tricyclo- [5.2.1.0²⁻ ⁶]décane 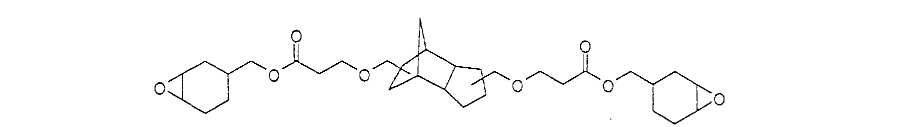
xi) bis(méthanediyloxy-3,1-propanediyl-1,1,3,3-0tétraméthyldisiloxanediyl-1,2-éthanediyl-3,4-époxycyclohexyl]tricyclo[5.2.1.0^{2,6}] décane 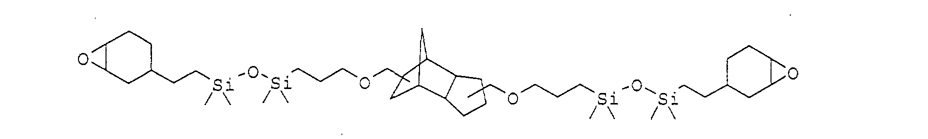
xii) bis{méthanediyloxy-3,1-propanediyl-3-oxatricyclo[3.2.1.0^{2,6}] octyl-6-carboxyl}tricyclo-[5.2.1.0^{2,6}]décane 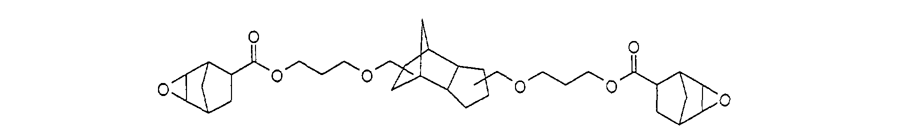
xiii) bis{méthanediyloxy-3,1-propanediyl-3,8-dioxa- tricyclo[3.2.1.0^{2,6}]octyl-6-carboxyl}tricyclo-[5.2.1.0^{2,6}]décane 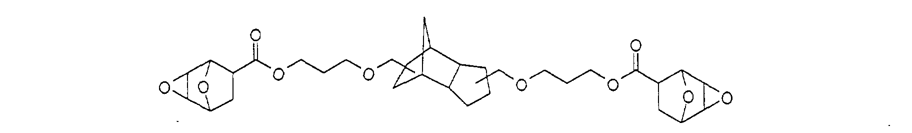
xiv) bis(méthanediyloxy-(3-propanediyl-3,5,7-tris-(2,1-éthanediyl-3,4-époxycyclohexyl)-1,3,5,7-tétraméthylcyclotétrasiloxanyl)-tricyclo-[5.2.1.0^{2,6}] décane 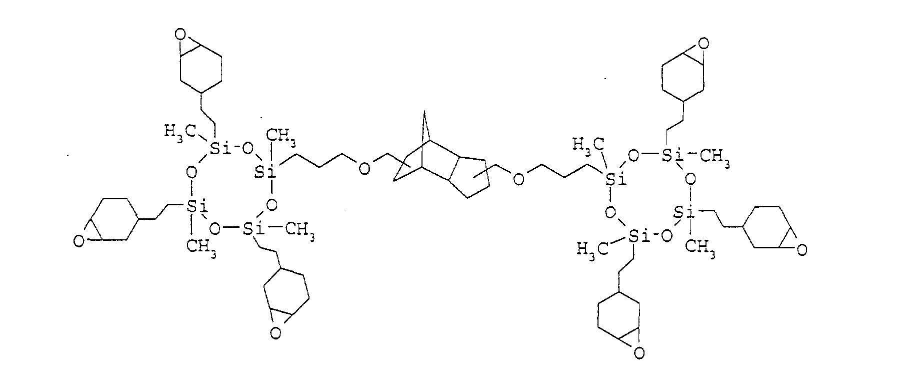
xv) 1,1,1-tris[méthanediyloxyméthanediyl-3,4-époxycyclohexyl]propane 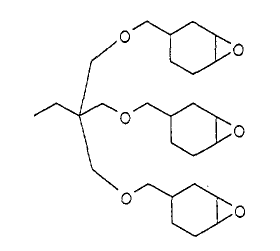
xvi) 1,1,1-tris[méthanediyloxy-1,3-propanediyl-1,1,3,3-tétraméthyldisiloxanediyl-1,2- éthanediyl-3,4-époxycyclohexyl]propane 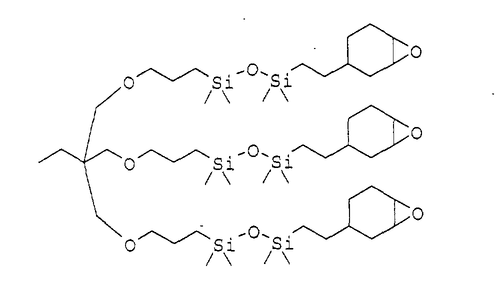
xvii) 1,1,1-tris{méthanediyl-3-oxatricyclo-[3.2.1.0^{2,4}]octyl-6-carboxy}propane 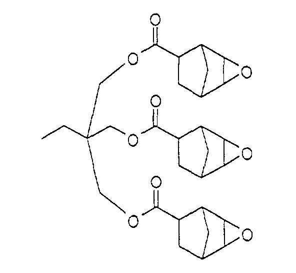
xviii) 1,1,1-tris{méthanediyl-3,8-dioxatricyclo- [3.2.1.0^{2,4}]octyl-6-carboxy}propane 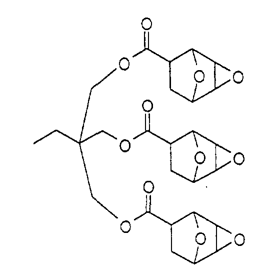
xix) 1,1,1-tris[méthanediyloxy-3,1-propanediyl-3,5,7-tris(2,1-éthanediyl-3,4-époxycyclohexyl)-1,3,5,7-tétraméthylcyclotétrasiloxanyl]propane 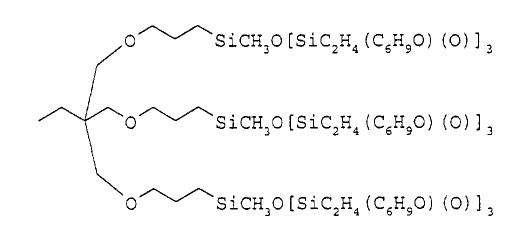
xx) 1,1,1-tris[méthanediyloxy-bis(éthanediyloxy)-3,4-époxycyclohexylcarboxylate]propane 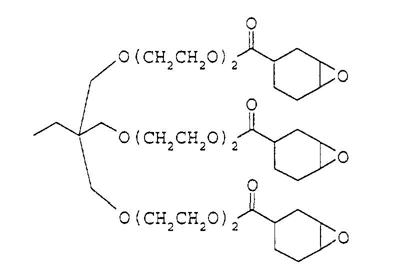
xxi) 1,1,1-tris[méthanediyloxy-bis(éthanediyloxy)-méthanediyl-3,4-époxycyclohexyl]propane 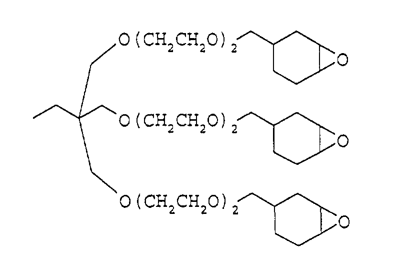
xxii) 1,1,1-tris[méthanediyloxybis(éthanediyloxy)-propanediyl-1,1,3,3-tétraméthyldisiloxanyl-1,2-éthanediyl-3,4-époxycyclohexyl]propane 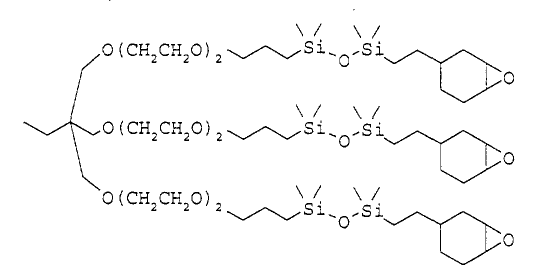
xxiii) 1,1,1-tris{méthanediyloxybis(éthanediyloxy)-3-oxatricyclo[3.2.1.0^{2,4})octyl-6-carboxy}propane 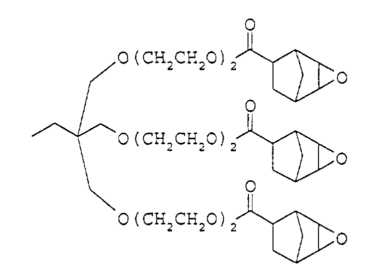
xxiv) 1,1,1-tris{méthanediyloxybis (éthanediyloxy)-3,8-dioxatricyclo[3.2.1.0^{2,4}]octyl-6-carboxy}-propane 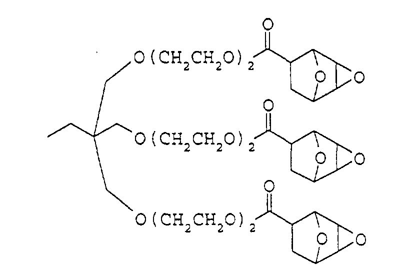
xxv) 1,1,1-tris[méthanediyloxy-bis(éthanediyloxy)-propanediyl-3,5,7-tris(2,1-éthanediyl-3,4-époxycyclohexyl)-1,3,5,7-tétraméthylcyclotétrasiloxanyl]propane 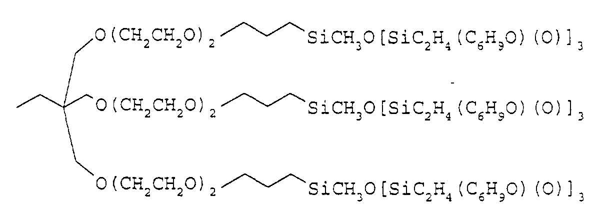
xxvi) α,ω-bis[3,4-époxycyclohexyléthanediyl-1,1,3,3- tétraméthyldisiloxanyl-3,1-propanediyl]polytétrahydrofurane 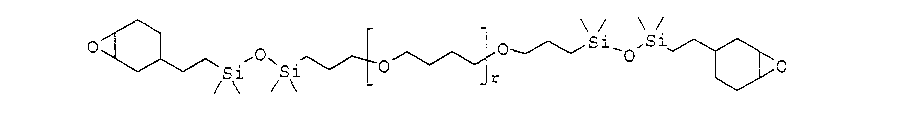
xxvii) α,ω-bis{3-oxatricyclo[3.2.1.0^{2,4}]octyl-6-carboxy}polytétrahydrofurane 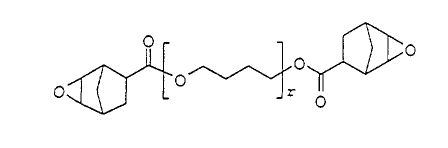
xxviii) α,ω-bis{3,8-dioxatricyclo[3.2.1.0^{2,4}]octyl-6-carboxy}polytétrahydrofurane 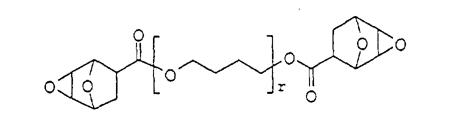
xxix) α,ω-bis(3-propanediyl-3,5,7-tris(2,1-éthane-diyl-3,4-époxycyclohexyl)-1,3,5,7-tétraméthylcyclotétrasiloxanyl)polytétrahydrofurane

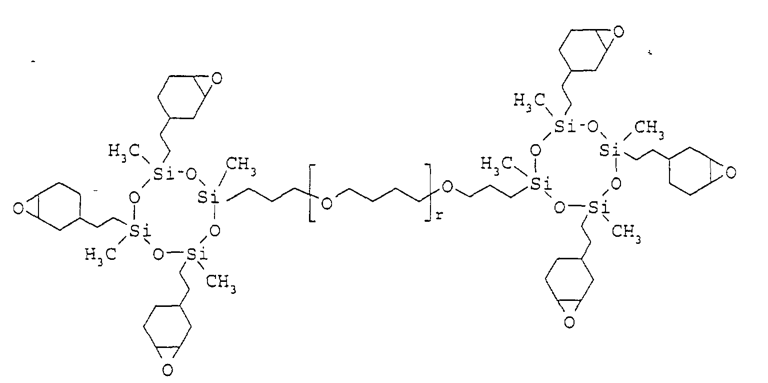

3. Composition polymérisable selon la revendication 1 ou la revendication 2, **caractérisée en ce qu'**elle contient comme époxyde de basse viscosité correspondant au composant b) du 3,4-époxycyclohexanecarboxylate de 3,4-époxycyclohexylméthyle, du 3,4-époxy-6-méthyl-cyclo hexanecarboxylate de 3,4-époxy-6-méthylcyclohexyle, du dioxyde de dicyclopentadiène, de l'adipate de bis(3,4-époxycyclohexylméthyle), du 1,3,5,7-tétrakis(2,1-éthanediyl-3,4-époxycyclohexyl)-1,3,5,7-tétraméthylcyclotétrasiloxane, du 1,3,5,7,9-pentakis(2,1-éthanediyl-3,S-époxycyclohexyl)-1,3,5,7,9-pentaméthylcyclopentasiloxane, du 1,1,3,3-tétraméthyl-1,3-bis(éthanediyl-3,4-époxycyclohexyl)disiloxane et/ou des siloxanes de faible masse moléculaire qui sont fonctionnalisés avec des époxydes cycloaliphatiques.

4. Composition polymérisable selon l'une au moins des revendications 1 à 3, **caractérisée en ce qu'**elle contient comme matières de charge correspondant au composant c) du quartz, des verres broyés, des gels de silice ou des acides siliciques ou des granulés de ceux-ci.

5. Composition polymérisable selon l'une au moins des revendications 1 à 4, **caractérisée en ce qu'**elle contient comme retardateurs, accélérateurs et/ou amorces des acides de Lewis ou des acides de Broensted ou des composés à partir desquels de tels acides sont dérivés par insolation avec de la lumière ultraviolette ou visible, par exposition à de la chaleur et/ou par application d'une pression.

6. Composition polymérisable selon l'une au moins des revendications 1 à 5, **caractérisée en ce qu'**elle contient comme adjuvants des diols, des diluants, des stabilisateurs, des inhibiteurs et/ou des pigments.

7. Composition polymérisable selon l'une au moins des revendications 1 à 6, composée de
A Une pâte de base contenant les époxydes correspondants aux composants (a) et (b), une partie ou la totalité de la matière de charge correspondant au composant (c), le cas échéant des retardateurs ou des accélérateurs correspondants au composant (d) et le cas échéant des adjuvants correspondants au composant (e), ainsi que, séparée dans l'espace de celle-ci
B une pâte catalysatrice contenant une amorce correspondant au composant (d), le cas échéant une partie de la matière de charge correspondant au composant (c) et le cas échéant des adjuvants correspondants au composant (e).

8. Epoxydes, à savoir
i) 2,2-bis[4,1-phénylénoxy-3,1-propanediyl-3,4-époxycyclohexylcarboxylate]propylidène 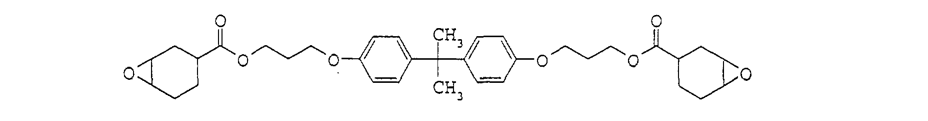
ii) 2,2-bis[4,1-phénylénoxy-3,1-propanediyloxyméthanediyl-3,4-époxycyclohexyl]propylidène 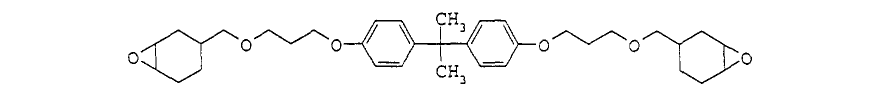
iii) 2,2-bis[3,4-époxycyclohexylméthanediyl(4,1-phénylénoxy-3,1-propylcarboxylate)]propylidène 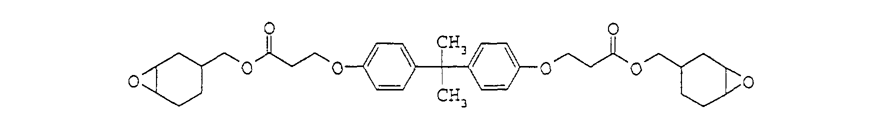
iv) 2,2-bis[4,1-phénylénoxy-3,1-propanediyl-1,1,3,3-tétraméthyldisiloxanyl-1,2-éthanediyl-3,4-époxycyclohexyl]propylidène 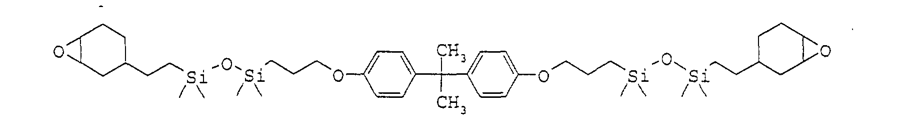
v) 2,2-bis{4,1-phénylénoxy-3,1-propanediyl-3-oxa-tricyclo[3.2.1.0^{2,4}]octyl-6-carboxy}propylidène 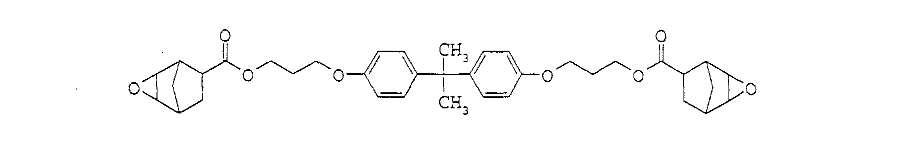
vi) 2,2-bis{4,1-phénylénoxy-3,1-propanediyl-3,8-dioxatricyclo[3.2.1.0^{2,4}]octyl-6-carboxy}-propylidène 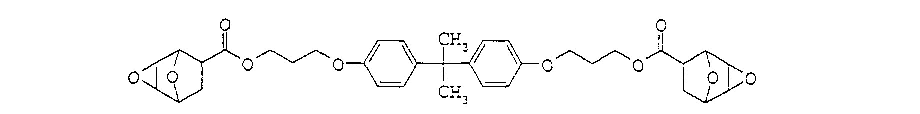
vii) 2,2-bis{4,1-phénylénoxy-3,1-propanediyl-[3,5,7- tris(éthanediyl-3,4-époxycyclohexyl)-1,3,5,7-tétraméthylcyclotétrasiloxanyl]}propylidène 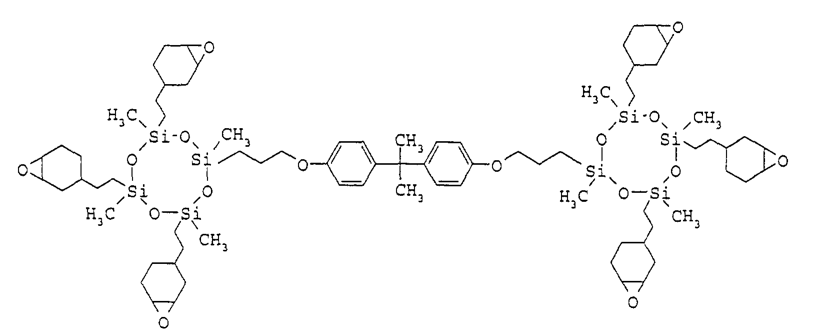
viii) bis[méthanediyloxy-3,1-propanediyl-3,4-époxy-cyclohexylcarboxylate]tricyclo[5.2.1.0^{2,6}]décane 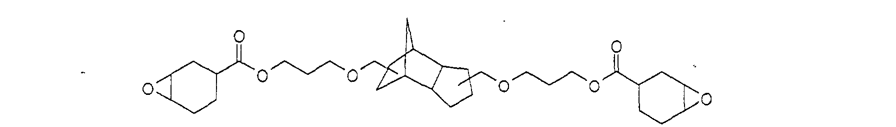
ix) bis[méthanediyloxy-3,1-propanediyloxyméthanediyl-3,4-époxycyclohexyl]tricyclo[5.2.1.0^{2,6}]-décane 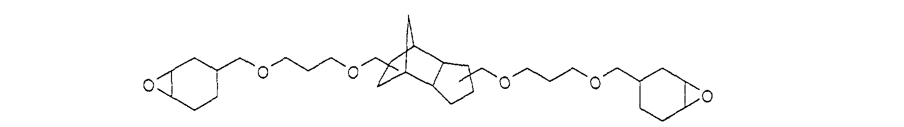
x) bis[3,4-époxycyclohexylméthanediylpropanecarboxylate-1-oxyméthanediyl]tricyclo-[5.2.1.0²⁻ ⁶]décane 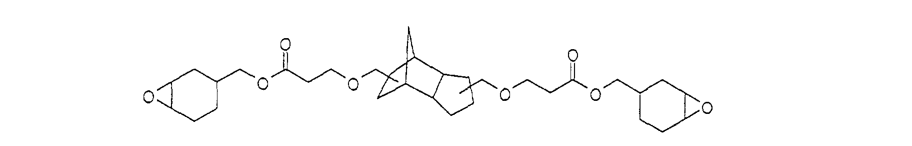
xi) bis(méthanediyloxy-3,1-propanediyl-1,1,3,3-tétraméthyldisiloxanediyl-1,2-éthanediyl-3,4-époxycyclohexyl]tricycle[5.2.1.0^{2,6}] décane 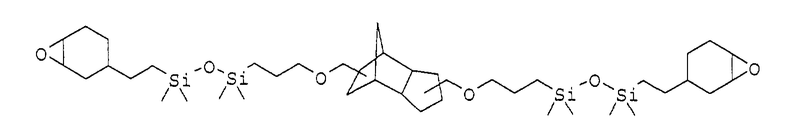
xii) bis{méthanediyloxy-3,1-propanediyl-3-oxatricyclo[3.2.1.0^{2,6}]octyl-6-carboxyl}tricyclo-[5.2.1.0^{2,6}] décane 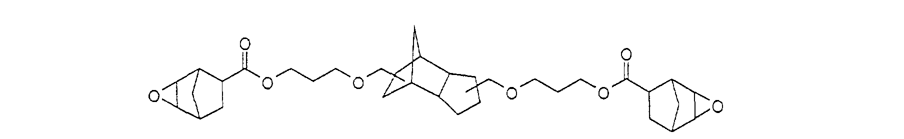
xiii) bis{méthanediyloxy-3,1-propanediyl-3,8-dioxa-tricyclo[3.2.1.0^{2,6}]octyl-6-carboxyl}tricyclo-[5.2.1.0^{2,6}] décane 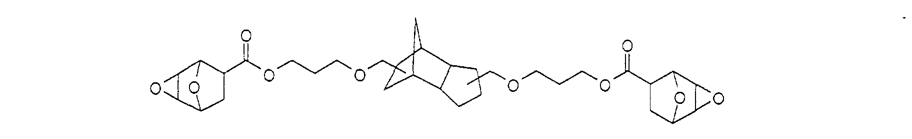
xiv) bis(méthanediyloxy-(3-propanediyl-3,5,7-tris-(2,1-éthanediyl-3,4-époxycyclohexyl)-1,3,5,7-tétraméthylcyclotétrasiloxanyl)-tricyclo-[5.2.1.0^{2,6}] décane 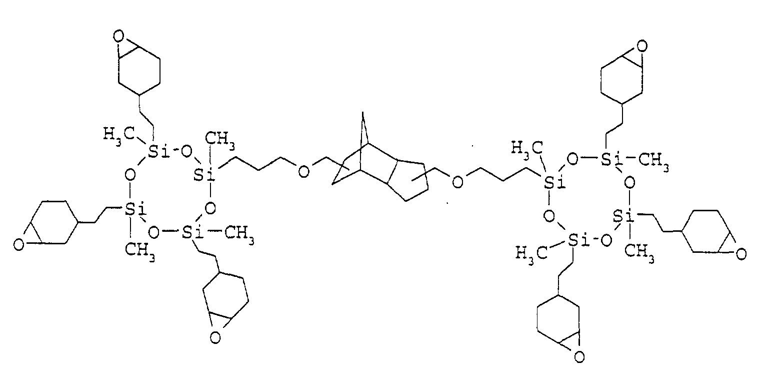
xv) 1,1,1-tris[méthanediyloxyméthanediyl-3,4-époxycyclohexyl]propane 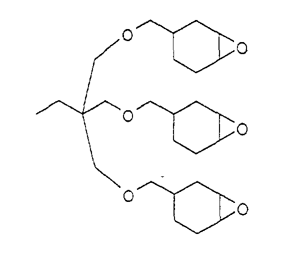
xvi) 1,1,1-tris[méthanediyloxy-1,3-propanediyl-1,1,3,3-tétraméthyldisiloxanediyl-1,2-éthanediyl-3,4-époxycyclohexyl]propane 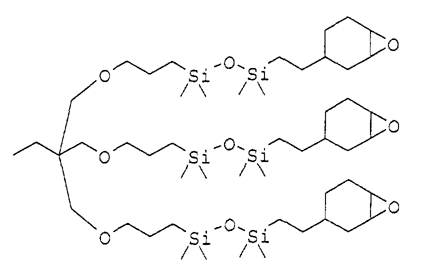
xvii) 1,1,1-tris{méthanediyl-3-oxatricyclo-[3.2.1.0^{2,4}] octyl-6-carboxy}propane 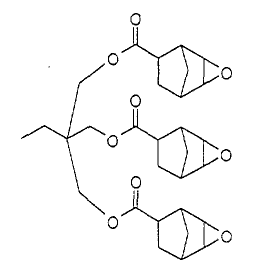
xviii) 1,1,1-tris{méthanediyl-3,8-dioxatricyclo-[3.2.1.0^{2,4}] octyl-6-carboxy}propane 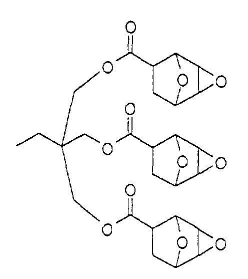
xix) 1,1,1-tris[méthanediyloxy-3,1-propanediyl-3,5,7-tris(2,1-éthanediyl-3,4-époxycyclohexyl)-1,3,5,7-tétraméthylcyclotétrasiloxanyl]propane 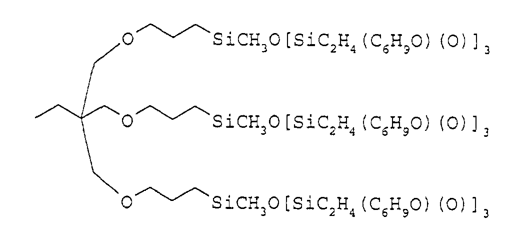
xx) 1,1,1-tris[méthanediyloxy-bis(éthanediyloxy)-3,4-époxycyclohexylcarboxylate]propane 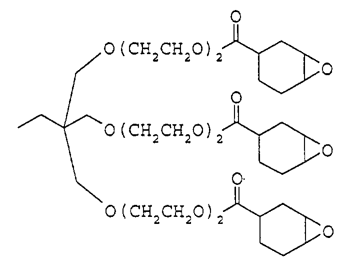
xxi) 1,1,1-tris[méthanediyloxy-bis(éthanediyloxy)-méthanediyl-3,4-époxycyclohexyl]propane 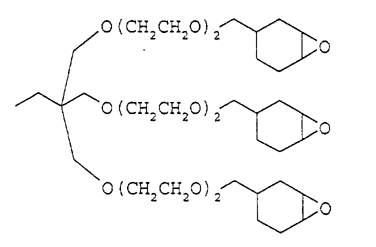
xxii) 1,1,1-tris[méthanediyloxybis(éthanediyloxy)-propanediyl-1,1,3,3-tétraméthyldisiloxanyl-1,2-éthanediyl-3,4-époxycyclohexyl]propane 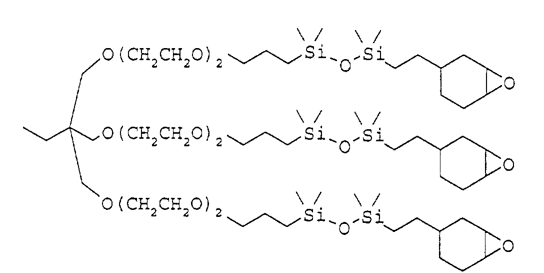
xxiii) 1,1,1-tris{méthanediyloxybis (éthanediyloxy)-3-oxatricyclo[3.2.1.0^{2,4}) octyl-6-carboxy}propane 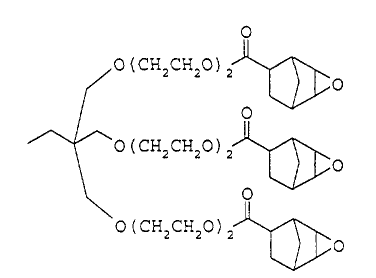
xxiv) 1,1,1-tris{méthanediyloxybis(éthanediyloxy)-3,8-dioxatricyclo[3.2.1.0^{2,4}]octyl-6-carboxy}-propane 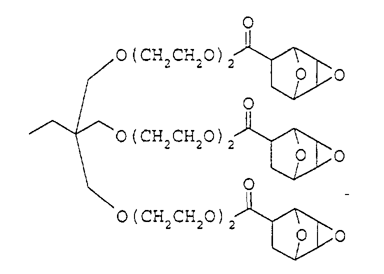
xxv) 1,1,1-tris[méthanediyloxy-bis(éthanediyloxy)-propanediyl-3,5,7-tris(2,1-éthanediyl-3,4-époxycyclohexyl)-1,3,5,7-tétraméthylcyclotétrasiloxanyl]propane 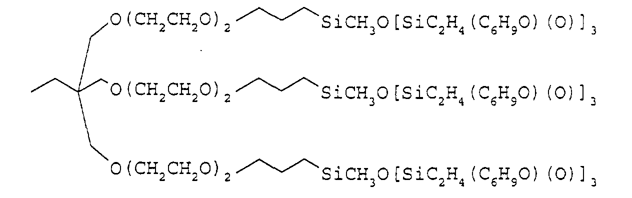
xxvi) α,ω-bis[3,4-époxycyclohexyléthanediyl-1,1,3,3-tétraméthyldisiloxanyl-3,1-propanediyl]polytétrahydrofurane 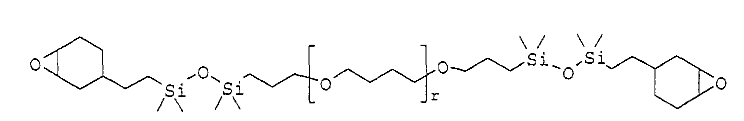
xxvii) α,ω-bis{3-oxatricyclo[3.2.1.0^{2,4}]octyl-6-carboxy}polytétrahydrofurane 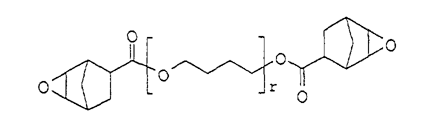
xxviii) α,ω-bis{3,8-dioxatricyclo[3.2.1.0^{2,4}]octyl-6-carboxy}polytétrahydrofurane 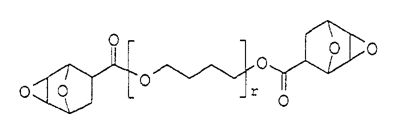
xxix) α,ω-bis(3-propanediyl-3,5,7-tris(2,1-éthane-diyl-3,4-époxycyclohexyl)-1,3,5,7-tétraméthylcyclotétrasiloxanyl)polytétrahydrofurane

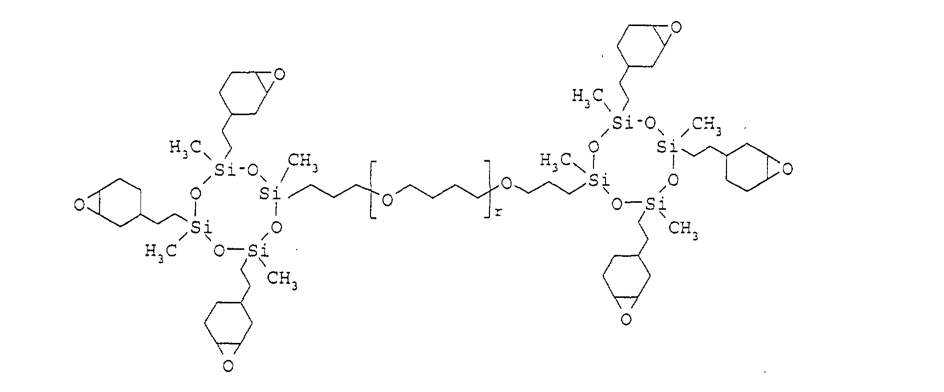

9. Utilisation de la composition polymérisable selon l'une au moins des revendications 1 à 7 ou des époxydes cycloaliphatiques selon la revendication 8 comme agents de revêtement.

10. Utilisation de la composition polymérisable selon l'une au moins des revendications 1 à 7 ou des époxydes cycloaliphatiques selon la revendication 8 pour le collage de substrats.

11. Utilisation de la composition polymérisable selon l'une au moins des revendications 1 à 7 ou des époxydes cycloaliphatiques selon la revendication 8 comme compositions à usage dentaire.
